(19) Europäisches Patentamt
European Patent Office
Office européen des brevets

(11) **EP 2 014 307 A2**

(12) **EUROPEAN PATENT APPLICATION**

(43) Date of publication:
**14.01.2009 Bulletin 2009/03**

(51) Int Cl.:
***A61K 47/48*** *(2006.01)* ***A61P 35/00*** *(2006.01)*
***A61P 25/00*** *(2006.01)* ***A61P 29/00*** *(2006.01)*

(21) Application number: **08018982.2**

(22) Date of filing: **13.03.2002**

(84) Designated Contracting States:
**AT BE CH CY DE DK ES FI FR GB GR IE IT LI LU MC NL PT SE TR**

(30) Priority: **13.03.2001 US 275725 P**
**07.11.2001 US 337935 P**

(62) Document number(s) of the earlier application(s) in accordance with Art. 76 EPC:
**08017419.6**
**02706567.1 / 1 418 945**

(71) Applicant: **ANGIOTECH INTERNATIONAL AG**
**63047 Zug (CH)**

(72) Inventors:
- **Liggins, Richard**
  **Coquitlam**
  **British Columbia V3J 5E8 (CA)**
- **Murphy, Larry**
  **Vancouver**
  **British Columbia V6H 1N1 (CA)**
- **Guan, Dechi**
  **Vancouver**
  **British Columbia V6P 3Y1 (CA)**

(74) Representative: **Barton, Matthew Thomas**
**Forrester & Boehmert**
**Pettenkoferstrasse 20-22**
**80336 München (DE)**

Remarks:
This application was filed on 30-10-2008 as a divisional application to the application mentioned under INID code 62.

(54) **Micellar drug delivery vehicles and uses thereof**

(57) Anhydrous formulations are formed of a diblock copolymer (X-Y) having a hydrophilic bloc X comprising residues of monomer x, and a hydrophobic block Y comprising residues of monomer y; an additive selected from a polymer and an organic solvent, where the solvent is water-miscible and biocompatible, and the polymer is hydrophobic or hydrophilic and comprises residues or monomers x and/or y, the polymer optionally having a molecular weight that is less than the molecular weight of the diblock copolymer; and a drug. Upon admixture with water, the anhydrous formulations form drug delivery vehicles, preferably in micellar form. The inclusion of polymer and/or organic solvent provides for the formation of micelles at an enhanced rate, and/or provides the formation of micelles having an enhanced ability to incorporate drug (s), and/or provides the formation of micelles having advantageous physical characteristics, *e.g.*, advantageous viscosity and/or melting point.

EP 2 014 307 A2

## Description

TECHNICAL FIELD

The present invention is related to drug delivery vehicles, more particularly to micellar drug delivery vehicles, to precursor compositions for drug delivery vehicles, and to methods of making such vehicles and precursors.

BACKGROUND OF THE INVENTION

Many pharmaceutically active compounds, i.e., drugs, intended for administration to a mammal, have limited solubility in water. This hydrophobic property often makes it difficult to formulate a drug so that it exhibits a satisfactory bioavailability profile *in vivo.* Poor bioavailability may lead to ineffective therapy, the need for higher dosing and/or undesirable side effects.

Delivery vehicles for hydrophobic pharmaceutically active compounds have been described. *See, e.g.,* U.S. Patent Nos. 6,096,338; 6,077,543; 5,843,891; 5,834,019; 5,827,541; 5,776,486; 5,645,856; 5,478,860; and 5,430,021. Micellar drug delivery systems have been used to deliver a hydrophobic drug to a subject (see, for example, Zhang, X. et al. International Journal of Pharmaceutics 132:195-206 (1996)).

There exists a need in the art for improved vehicles for the delivery of hydrophobic drugs, and for methods of forming improved vehicles.

SUMMARY OF THE INVENTION

The present invention provides improved, drug-containing compositions that may be combined with an aqueous medium to form a macroscopically homogeneous, fluid mixture wherein the drug is dispersed throughout the mixture, typically within micelles. The compositions of the present invention are particularly advantageous in that they may form micelles at an enhanced rate, have an enhanced ability to incorporate drug(s); and/or have advantageous physical characteristics, e.g., viscosity and/or melting point characteristics that render the compositions particularly easy to make and/or handle. The present invention also provides precursors to these compositions, methods to make the precursors and/or compositions, and other related compositions and methods as described below.

In one aspect, the present invention provides a composition comprising:

(a) a micelle-forming biocompatible diblock copolymer (X-Y) having a hydrophilic block X comprising residues of monomer x, and a hydrophobic block Y comprising residues of monomer y;
(b) an additive comprising at least one of a polymer or a water soluble, biocompatible, organic solvent; and
(c) a hydrophobic drug;

with the proviso that the composition forms a micellar solution. Preferably, the composition does not form a hydrogel upon combination of the composition with aqueous media.

In another aspect, the present invention provides a composition comprising:

(a) a micelle-forming biocompatible diblock copolymer (X-Y) having a hydrophilic block X comprising residues of monomer x, and a hydrophobic block Y comprising residues of monomer y;
(b) an additive comprising at least one of a polymer and an organic solvent,

  i) the solvent being water-soluble and biocompatible;
  ii) the polymer comprising monomer residues x and/or y; and

(c) a hydrophobic drug;

with the proviso that the composition forms a micellar solution in aqueous media. Optionally, the additive has a molecular weight that is less than the molecular weight of the diblock copolymer.

In another aspect, the present invention provides a composition comprising:

(a) a biocompatible diblock copolymer (X-Y) having a block X comprising residues of monomer x, and a block Y comprising residues of monomer y, the block X being more hydrophilic than the block Y;
(b) a biocompatible water-soluble additive comprising at least one of a polymer and an organic solvent; and
(c) a hydrophobic drug;

with the proviso that the composition forms a micellar solution in aqueous media.

In another aspect, the present invention provides a composition comprising:

(a) a biocompatible diblock copolymer (X-Y) having a hydrophilic block X and a hydrophobic block Y;
(b) a water-soluble biocompatible organic solvent,
(c) a hydrophobic drug; and
(d) water;

the composition comprising micelles.

In another aspect, the present invention provides a composition comprising:

(a) a biocompatible diblock copolymer (X-Y) having a hydrophilic block X, and a hydrophobic block Y;
(b) a hydrophilic polymer;
(c) a hydrophobic drug; and
(d) water;

the composition comprising micelles. Optionally, the hydrophilic polymer (b) has a number average molecular weight that is less than the number average molecular weight of the diblock copolymer (a).

In another aspect, the present invention provides a composition comprising:

(a) a biocompatible diblock copolymer (X-Y) having a hydrophilic block X, and a hydrophobic block Y;
(b) a hydrophobic polymer;
(c) a hydrophobic drug; and
(d) water;

the composition comprising micelles. Optionally, the hydrophobic polymer (b) has a number average molecular weight that is less than the number average molecular weight of the diblock copolymer (a).

In another aspect, the present invention provides a sterile composition that is packaged within a container that maintains the sterility of the composition for a sufficient time to be useful, e.g., one week.

In another aspect, the present invention provides a method for forming a drug delivery vehicle, comprising sequentially providing a non-aqueous composition as described herein; and adding aqueous media to the composition to form a micelle-containing composition.

In another aspect, the present invention provides a method of forming a composition comprising combining, and preferably dissolving, a hydrophobic drug with/in an additive and then adding diblock copolymer to the additive, where the hydrophobic drug, additive and diblock copolymer are described herein.

In another aspect, the present invention provides a method for preparing a composition, the method comprising

(a) dissolving a micelle-forming biocompatible diblock copolymer (X-Y) having a hydrophilic block X comprising residues of monomer x, and a hydrophobic block Y comprising residues of monomer y in a purification solvent,
(b) precipitating or crystallizing the diblock copolymer from the purification solvent; and
(c) separating the diblock copolymer of (b) from the purification solvent.

In another aspect, the present invention provides a method for preparing a composition, the method comprising

(a) dissolving a micelle-forming biocompatible diblock copolymer (X-Y) having a hydrophilic block X comprising residues of monomer x, and a hydrophobic block Y comprising residues of monomer y in a purification solvent,
(b) separating the purification solvent from the diblock copolymer;
(c) repeating (a); and
(d) repeating (b).

In another aspect, the present invention provides a method for preparing a composition, the method comprising

(a) dissolving a micelle-forming biocompatible diblock copolymer (X-Y) having a hydrophilic block X comprising residues of monomer x, and a hydrophobic block Y comprising residues of monomer y in a purification solvent,
(b) adding activated charcoal to (a);
(c) filtering the activated charcoal from (b);
(d) removing the purification solvent from the copolymer.

In another aspect, the present invention provides a method of treating a disease in a mammal comprising the administration of an effective amount of a composition of the present invention to said mammal. Optionally, the composition includes aqueous media, e.g., pure water, and also optionally the composition includes micelles.

In another aspect, the present invention provides a method of preventing a disease in a mammal comprising the administration of an effective amount of a composition of the present invention to said mammal. Optionally, the composition includes aqueous media, e.g., pure water, and also optionally the composition includes micelles.

These and other aspects of the present invention will become evident upon reference to the following detailed description. In addition, various references are set forth herein. Each of these references is incorporated herein by reference in its entirety as if each were individually noted for incorporation. These references include the following: U.S. Patent Application Nos. 60/032,215; 60/063,087; 60/275,725; 60/288,017; /337,935; 08/094,536; 08/417,160; 08/480,260; 08/486,867; 08/980,549; 08/984,258; 09/013,765; 09/088,546; 09/368,871; 09/201,695; 09/294,458; and 09/368,463; U.S. Patent Nos. 5,716,981; 5,886,026; and 5,994,341; European Patent Application Nos. EP 96119361.2; 97945697.7; 00123557.1; 00123534.0; and 00123537.3; European Patent No. 0 706 376; and PCT Patent Application Nos. PCT/CA94/00373; PCT/CA97/00910; and PCT/CA99/00464.

## DETAILED DESCRIPTION OF THE INVENTION

In one aspect, the present invention provides hydrophobic drug-containing compositions that, upon combination with aqueous media, e.g., pure water or aqueous buffer, provide a micelle-containing composition. The hydrophobic drug, which is not very soluble in water alone, is effectively solubilized in a micelle-containing composition according to the present invention.

For instance, in one aspect the present invention provides a composition that includes: (a) a micelle-forming biocompatible diblock copolymer (X-Y) having a hydrophilic block X comprising residues of monomer x, and a hydrophobic block Y comprising residues of monomer y; (b) an additive selected from a polymer and a water soluble, biocompatible, organic solvent; and (c) a hydrophobic drug. The composition forms a micelle-containing solution, also known as a micellar solution, when combined with aqueous media. The composition preferably does not form any hydrogel when combined with aqueous media.

In general, the term "includes" as used above and elsewhere herein is intended to denote that the composition may, but need not, contain components not within the scope of the specifically enumerated components, which in the case of the above-described composition are components (a), (b), and (c). In various additional aspects of the present invention, the term "includes" when used herein to describe a composition may be replaced with the term "includes only", where the term "includes only" is intended to denote that the composition contains only the enumerated components and no other components. It should be understood that the terms "a" and "an" as used above and elsewhere herein refer to "one or more" of the enumerated components. Thus, the composition described above is intended to describe compositions that contain one or more chemically distinct diblock copolymers and/or one or more chemically distinct additives and/or one or more hydrophobic drugs.

Another specific example of a composition of the present invention is a composition that includes: (a) a micelle-forming biocompatible diblock copolymer (X-Y) having a hydrophilic block X comprising residues of monomer x, and a hydrophobic block Y comprising residues of monomer y; (b) an additive selected from a polymer and an organic solvent, where i) the solvent is water-soluble and biocompatible; and ii) the polymer comprises monomer residues x and/or y; and (c) a hydrophobic drug. In one aspect, the composition forms a micellar solution when combined with aqueous media. The composition preferably does not form any hydrogel when combined with aqueous media. In one embodiment, the polymer has a number average molecular weight that is less than the number average molecular weight of the diblock copolymer.

As another specific example, the present invention provides a composition that includes: (a) a biocompatible diblock copolymer (X-Y) having a block X comprising residues of monomer x, and a block Y comprising residues of monomer y, the block X being more hydrophilic than the block Y; (b) a biocompatible water-soluble additive selected from a polymer and an organic solvent, and (c) a hydrophobic drug. In one aspect, the composition forms a micellar solution when combined with aqueous media. The composition preferably does not form any hydrogel when combined with aqueous media. In one embodiment, the additive has a molecular weight that is less than the molecular weight of the copolymer.

As used herein, the term "comprises residues of monomer x" is used in the context of describing a polymer or copolymer. As is well known to one of ordinary skill in the art, polymers and copolymers are typically formed by the polymerization of monomers. In the formation of a polymer or copolymer, a monomer will react with a growing polymer or copolymer chain, so as to both join the chain and also form a reactive site to which the next monomer may join. As this process repetitively occurs, the polymer or copolymer chain grows to its final length. The monomer is structurally changed by its incorporation into a polymer or copolymer, and the resulting structure is referred to herein as the residue of the monomer. Thus, a polymer or copolymer may be viewed as a chain of monomer residues.

As used herein, a "block" copolymer is a copolymer having distinct structural regions, i.e., subunit regions that are structurally distinct from one another. A subunit region is a series (chain) of monomer residues, as defined above. A diblock copolymer has two distinct structural regions, where the subunit composition in one block differs from the subunit composition in the second block. For instance, a diblock copolymer may be formed from a block (series, chain) of acrylic acid residues adjacent to a block of methacrylic acid residues. Another examples of a diblock copolymer is a block of acrylic acid residues adjacent to a block formed by a mixture of ethylene oxide and propylene oxide residues.

The block copolymers utilized in the invention will preferably form micelles in isotonic aqueous solutions at a physiological temperature, and the micelles will have diameters within the range of about 1 nm to about 100 nm. In various embodiments, the micelles have an average diameter of 1-100, 1-90, or 1-80, or 1-70, or 1-60, or 1-50, or 1-40, or 1-30, or 1-20, or 5-100, or 5-90, or 5-80, or 5-70, or 5-60, or 5-50, or 5-40, or 5-30, or 5-20, or 10-100, or 10-90, or 10-80, or 10-70, or 10-60, or 10-50, or 10-40, or 10-30, or 10-20 nm. In a preferred embodiment, the micelles have an average diameter of about 15 nm. The blocks have "hydrophobic" and "hydrophilic" characters that are sufficiently hydrophobic and hydrophilic, respectively, to provide an amphiphilic molecule that can form a micelle in an aqueous media.

The term "biocompatible" is commonly used in the art, and is used herein according to its art-recognized meaning. For further clarity, it can be noted that a "biocompatible" material is one that does not illicit undue toxicity, irritancy, foreign body response or inflammation when it is contacted with an animal. If the biocompatible material degrades in the host, the degradation products are biocompatible degradation products.

The diblock copolymer is not only preferably biocompatible, but it is also preferably biodegradable. Thus, in one aspect of the invention, the diblock copolymer is both biocompatible and biodegradable.

As used herein, the term micelle has its ordinary and accustomed meaning as understood by one of ordinary skill in the art, and thus refers to a noncovalently associated collection (aggregate) of many simple molecules that together function as a unit having unique properties (e.g., aqueous solubilization of water-insoluble materials) that are not observed with the individual molecules which comprise the micelle. The micelles of the present invention are supramolecular complexes comprising diblock copolymer, where the micelles form in aqueous solutions due to microphase separation of the nonpolar portions of the copolymers. Micelles form when the concentration of the diblock copolymer reaches, for a given temperature, a critical micelle concentration (CMC) that is characteristic of the copolymer. As referred to herein, a micelle is not necessarily spherical, but may assume other shapes, e.g., rod-shaped or laminar.

By varying the sizes of the hydrophilic and hydrophobic segments of the block copolymers, the tendency of the copolymers to form micelles at physiological conditions, as well as the average size of the micelles formed at the physiological conditions, can be varied. These tendencies can also be influenced by blending copolymers with differing mixes of hydrophobic and hydrophilic blocks. The micelles have a dense core formed by the water-insoluble repeating units of the Y blocks and lipophilic portions of a biological agent dissolved therein, and a hydrophilic shell formed by the X blocks and hydrophilic portions (if any) of the biological agent. The micelles have translational and rotational freedom in aqueous environment, and aqueous environments containing the micelles have low viscosity similar to water. Micelle formation typically occurs at copolymer concentrations from about 0.001 to 5% (w/v), or 0.001 to 1% (w/v), or about 0.005 to 0.5% (w/v). Here and elsewhere herein the term x% (w/v) indicates a weight of copolymer as measured in grams per volume of aqueous solution as measured in a unit of 100 milliliters. Thus, 1% (w/v) refers to 1 grams dissolved in 100 mL of solvent.

In the compositions of the present invention, the block X of the block copolymer comprises residues of one or more monomers selected from (meth)acrylic acid, vinylpyrrolidone, saccharide, and amino acid. As used herein (meth) acrylic acid refers both to acrylic acid and methacrylic acid. Suitable saccharides that may be a monomer for block X include, without limitation, mono-, di- and trisaccharides. Preferred saccharides are glucose, mannose, fructose, sucrose, lactose, maltose, trehalose and raffinose. Amino acids have both an amine group and a carboxylic acid group, where suitable amino acids that may be a monomer for block X include, without limitation, naturally and non-naturally occurring amino acids. The preferred naturally occurring amino acids for use in the present invention are alanine, arginine, asparagine, aspartic acid, citrulline, cysteine, cystine, glutamine, glycine, histidine, isoleucine, leucine, lysine, methionine, ornithine, phyenylalanine, proline, serine, threonine, tryptophen, tyrosine, valine, hydroxy proline, γ-carboxyglutamate, phenylglycine, or O-phosphoserine. The preferred non-naturally occurring amino acids for use in the present invention are β-alanine, α-amino butyric acid, γ-amino butyric acid, γ-(aminophenyl) butyric acid, α-amino isobutyric acid, ε-amino caproic acid, 7-amino heptanoic acid, β-aspartic acid, aminobenzoic acid, aminophenyl acetic acid, aminophenyl butyric acid, γ-glutamic acid, cysteine (ACM), ε-lysine, ε-lysine, (A-Fmoc), methionine sulfone, norleucine, norvaline, ornithine, d-ornithine, p-nitro-phenylalanine, hydroxy proline, 1,2,3,4,-tetrahydroisoquinoline-3-carboxylic acid and thioproline.

In one aspect of the invention, block X of the block copolymer comprises residues of alkylene oxide. Suitable alkylene oxides include ethylene oxide, propylene oxide and butylene oxide.

In one aspect, the block copolymer comprises adjacent repeating units of the residue from acrylic acid, or adjacent repeating units of the residue from vinyl pyrrolidone, or adjacent repeating units of the residue from saccharide, or adjacent repeating units of the residue from amino acid, so that block X comprises poly(acrylic acid), poly(vinyl pyrrolidone), poly(saccharide), or poly(amino acid), respectively. In various aspects of the present invention, block X

comprises poly(acrylic acid), poly(vinyl pyrrolidone), poly(saccharide), or poly(amino acid) as the sole polymeric material within block X.

In another aspect, the block copolymer comprises adjacent repeating units of the residue from alkylene oxide (s), so that block X comprises poly(alkylene oxide) as the sole polymeric material within block X. In an optional embodiment, the poly(alkylene oxide) is selected from poly(ethylene oxide) and terminal $C_1$-$C_6$ alkyl ethers of poly(ethylene oxide). As used herein, the term "$C_1$-$C_6$" refers to a moiety having from 1 to 6 carbons, *i.e.,* having 1, 2, 3, 4, 5 or 6 carbons. A preferred terminal $C_1$-$C_6$ alkyl ether of poly(ethylene oxide) is a $C_1$ alkyl ether of poly(ethylene oxide), also known as MePEG. MePEG comprises a preferred X block of the present invention.

In one aspect, the block Y comprises residues of monomers selected from methacrylic acid, esters of methacrylic acid, esters of acrylic acid, and vinyl acetate. Suitable esters include alkyl esters (e.g., methyl ester, ethyl ester, propyl ester). In another aspect, the block Y comprises residues of monomers selected from lactic acid and reactive equivalents thereof, glycolic acid and reactive equivalents thereof, and caprylic acid and reactive equivalents thereof. Reactive equivalents of lactic acid include: D-lactic acid, L-lactic acid, DL-lactic acid, DL-lactide, L-lactide and D-lactide. Reactive equivalents of glycolic acid include glycolide. Reactive equivalents of caprylic acid include: caprolactone, valerolactone, and butyrolactone.

In one aspect of the present invention, the block Y is selected from polylactide, polyglycolide, polycaprolactone, hydrophobic polypeptides, hydrophobic polycarbonates, poly(vinyl acetate) and copolymers thereof. In one aspect, the block Y of the copolymer is poly-DL-lactide-co-glycolide, while in another aspect the block Y is poly-DL-lactide.

In a preferred aspect, the block X comprises residues of monomers selected from alkylene oxide, acrylic acid, vinyl pyrrolidone, saccharide, and amino acid, while the block Y comprises residues of monomers selected from lactide or reactive equivalents thereof, glycolide or reactive equivalents thereof, caprolactone or reactive equivalents thereof, hydrophobic amino acid, carbonate, and vinyl acetate. For example, in one aspect, block X comprises residues of monomers selected from alkylene oxide(s) while Y comprises residues of monomers selected from lactide or reactive equivalents thereof and glycolide or reactive equivalents thereof. As another example, block X comprises residues of ethylene oxide while block Y comprises residues of lactide. As yet another example, block X is MePEG and block Y is poly(DL-lactide).

The relative amounts, on a weight basis, of the blocks X and Y in the diblock copolymer is preferably controlled to allow the block copolymer to form a micelle in aqueous media. In one aspect, 100 parts of diblock copolymer comprises 30-90 parts hydrophilic polymer X and 60-10 parts hydrophobic polymer Y, where these parts are on a weight basis. In another aspect, 100 parts of diblock copolymer comprise 40-80 parts hydrophilic polymer X and 60-20 parts hydrophobic polymer Y. In yet another aspect, 100 parts of diblock copolymer comprise 50-70 parts hydrophilic polymer X and 50-30 parts hydrophobic polymer Y. In still another aspect, 100 parts of diblock copolymer comprise about 60 parts hydrophilic polymer X and about 40 parts hydrophobic polymer Y.

The relative amounts, on a weight basis, of the blocks X and Y in the diblock copolymer can be controlled in the following manner: a stoichiometric ratio of X:Y, x:y, X:y or x:Y may be combined as reagents in the reaction to produce the diblock copolymer. The result is a polymer having the composition of X:Y. *See, e.g.,* Zhang, X. et al. International Journal of Pharmaceutics 132:195-206 (1996).

The molecular weight of the diblock copolymer, in terms of number average molecular weight, is preferably controlled in order that that the diblock copolymer may form a micelle in aqueous media. In one aspect, the diblock copolymer has a number average molecular weight of about 1,000 to about 10,000 g/mol. In another aspect, the diblock copolymer has a number average molecular weight of about 2,000 to about 5,000 g/mol. In yet another aspect, the diblock copolymer has a number average molecular weight of about 2,500 to about 3,500 g/mol.

The molecular weight of the diblock copolymer containing the blocks X and Y can be controlled by selecting appropriate reaction conditions. By example, for the preparation of a diblock copolymer wherein X is polyalkylene oxide (from MePEG) and Y is poly-DL-lactide, the molecular weight is controlled by selecting a specific molecular weight of MePEG (block X) as a starting material and a specific ratio of X:y (where y is DL-lactide, the other starting material). In this synthesis the molecular weight is expected to be equal to:

$$\text{diblock copolymer molecular weight} = \text{molecular weight of X} + \text{mass of y/mass of X} * \text{molecular weight of X}$$

*See, e.g.,* Zhang, X. et al. International Journal of Pharmaceutics 132:195-206 (1996).

In one aspect of the invention, a purification process follows the preparation of the diblock copolymer. Typically, after preparation of the diblock copolymer, the product mixture will contain some unreacted starting materials and/or some by-products, i.e., reaction products that are other than the desired reaction products. In the synthesis of diblock copolymer comprising a methoxypolyethylene glycol block and a poly(DL-lactide) block, the by-products and/or unreacted starting material(s) may be residual monomer and other components that are acidic in nature. In this polymer the residual

monomer is DL-lactide. For diblock copolymers in which the hydrophobic block is a polyester, acidic by-products are anticipated to form, however their exact composition will vary depending on the monomer used.

Desirable properties for a copolymer include a limited amount of by-products or residual monomers from synthesis. The desirability of reduced by-products and/or residual monomer(s) contamination has been demonstrated by experimentation, and is a novel feature of the present invention. In one aspect, the diblock copolymer is 80% pure, which means that in one aspect the invention provides a composition comprising the diblock copolymer where at least 80% of the weight of the composition is contributed by the diblock copolymer. In other aspects of the invention, the diblock copolymer is 85% pure, 90% pure, 92% pure, 94% pure, 95% pure, 96% pure, 97% pure, 98% pure, 99% pure, 99.5% pure, or 99.9% pure.

A diblock copolymer useful in forming micelles according to the present invention may be synthesized according to methods disclosed in publications such as Zhang et al, 1996. Whether formed according to Zhang et al. or by some other procedure, the diblock copolymer may, as part of the synthesis process or at some later time, be exposed to organic solvents and/or activated carbon. It will typically be desirable to separate the diblock copolymer from the organic solvents and/or activated carbon. In one aspect of the invention, the diblock copolymer, as well as any starting materials and/or by-products that are in admixture with the diblock copolymer, is dissolved in an organic solvent and combined with activated carbon (also referred to herein as activated charcoal). After the activated carbon has had an opportunity to interact with the copolymer-containing composition, which occurs in a timeframe on the order or 10-60 minutes, the carbon is removed from the copolymer in a manner that allows starting materials and/or by-products that interact with the carbon, to be removed with the carbon.

For example, the copolymer may be dissolved in an organic purification solvent, where dissolution may involve heating up to about 55°C. The copolymer concentration in the solvent may be up to 50% on a weight basis, but is preferably less than 10%, less than 5% or less than or equal to 2.5%. The organic solvent may contain small amounts of water, preferably less than 20%, less than 10%, less than 5% or less than 2% of the total solvent volume. Suitable organic purification solvents include, but are not limited to, dichloromethane, ethanol, isopropanol, tetrahydrofuran or chloroform. Activated charcoal is added to this solution with mixing. After mixing, the activated charcoal is removed by means such as centrifugation or filtration. The resulting solution is then subjected to means that remove the solvent from the copolymer. For instance, the solvent may be removed by means such as drying under increased heat, and/or under vacuum or forced air or a dry gas such as nitrogen. Spray drying and freeze drying are two solvent-removal methods according to the present invention. Labconco, Kansas City, MI, is one supplier of freeze dryer systems and solvent evaporation systems. The use of a vacuum oven is a preferred option for removing solvent, where numerous suppliers of vacuum ovens are known to one of ordinary skill in the art, see, e.g., Binder GmbH, Germany; and M. Braun Inc., Stratham, N.H.

As another example, the copolymer may be dissolved in an organic solvent where the solvent and copolymer/solvent concentration are selected such that the copolymer is soluble in the solvent at elevated temperatures, but insoluble or partially insoluble at reduced temperatures. In other words, the copolymer can be crystallized or precipitated from the solvent. Isopropanol is a suitable solvent for this purpose. Mixing the copolymer with the solvent may involve heating in order to facilitate dissolution of the copolymer. In the case of isopropanol, a temperature of up to about 55°C is suitable. After mixing, the solution is cooled to facilitate precipitation of the copolymer from the solvent. The cooling temperature may be as low as about -20°C, but temperatures as high as 2-8°C are suitable for some solvents, such as isopropanol. After precipitation, the copolymer can be isolated from the solvent by, for example, filtration, and further dried if necessary by, for example, exposure to reduced pressure and/or elevated temperature that will encourage evaporation of the solvent. This process may be repeated as many times as necessary to achieve a copolymer with the desired properties. In one aspect, the process is repeated once. In another aspect the process is repeated twice.

The desirability of removing unreacted starting materials and/or acidic reaction by-products can be seen by reference to the data in Table 1. The data in Table 1 demonstrate that when acidic oligomers of DL-lactide and/or DL-lactide itself are added to a diblock copolymer or polyethylene glycol polymer containing little or none of these components, polymer matrices are produced that incorporate paclitaxel into the matrix with varying amounts of paclitaxel loss in the process. Addition of paclitaxel to the polymer matrices resulted in a reduction of paclitaxel content from the amount added and these reductions correspond to matrices containing elevated levels of the acidic species and residual monomer. According to the data, when paclitaxel is combined with a diblock copolymer having up to 2% residual monomer and up to 0.2 μmol/mg acid content (expressed as the number of protons titrated in a solution prepared by dissolving 1 mg of diblock copolymer in water), after heating, only 95% of the paclitaxel initially added to the mixture could be recovered, as quantified using a reverse phase HPLC assay with UV detection and a taxane optimized C18 column. Furthermore, in mixtures in which the diblock copolymer had greatly reduced quantities of the residual monomer and acidic components, 98% of the paclitaxel could be recovered and when paclitaxel was combined with methoxypolyethylene glycol having no measured acidic components and no DL-lactide monomer, 99% was recovered. Addition of DL-lactide and an acidic component resulted in further loss, compared to samples to which neither of these components were added.

Table 1

RECOVERY OF PACLITAXEL ADDED TO SEVEN DIFFERENT MIXTURES AFTER HEATING EACH OF THE MIXTURES TO 55°C FOR 1 HOUR

| Selected components contained in the mixture | Mixture number (X denotes the component is present in the mixture) | | | | | | |
|---|---|---|---|---|---|---|---|
| | 1 | 2 | 3 | 4 | 5 | 6 | 7 |
| ethoxy polyethylene glycol | | X | X | | | | |
| Diblock copolymer (not more than 2% residual monomer and 0.2 μmol of protons/mg in aqueous solution) | | | X | | | | |
| Diblock copolymer (less than 0.5% residual monomer and 0.05 μmol of protons/mg in aqueous solution) | | | | X | X | X | X |
| DL-lactide monomer (2%) | | | | | X | | X |
| DL-lactide oligomer (having 4 μmol of protons/mg in aqueous solution) | | | | | | X | X |
| Recovery of paclitaxel after heating (% of amount added to mixture) | 100 | 99 | 95 | 98 | 95 | 95 | 96 |

Based on these data, preferred diblock copolymers according to the present invention have the following compositional restrictions. In one aspect, the diblock copolymers comprising a polyalkylene oxide block and a polyester block have less than 5% residual monomer (from polyester starting material) and less than 0.4 μmol/mg acid (by an aqueous titration method). More preferred are limits of 2% residual monomer and 0.2 μmol/mg acid. Even more preferred are limits of 1% residual monomer and 0.05 μmol/mg acid. Still more preferred are limits of 0.5% residual monomer and

0.025 μmol/mg acid. The disclosed limits of these two components may be applied independent of one another. For instance, in one aspect the diblock copolymer is in combination with less than 5% residue monomer, and less than 0.025 μmol/mg acid. Every other combination of % residue monomer and upper limit of μmol/mg acid value as set forth above are provided according to various aspects of the present invention.

In another aspect of purification, precipitation from a solvent can lighten the color of the copolymer as a result of removing constituents and/or byproducts of the reaction which absorb light in the range of 300 to 500 nm, with a maximum absorbance at 315 nm, and a significant absorbance in many batches of diblock copolymer at 450 nm. After purification the copolymer is characterized by absorbance characteristics in the Table 2.

Table 2

LIGHTENING COLOR OF DIBLOCK COPOLYMER

| Batch # | Absorbance Values (AU) At: | | |
|---|---|---|---|
| | 315 nm | 425 nm | 450 nm |
| 1 unpurified | 2.58 | 0.464 | 0.262 |
| 1 purified | 0.442 | 0.137 | 0.100 |
| 2 unpurified | 2.39 | 0.264 | 0.136 |
| 2 purified | 0.178 | 0.0438 | 0.0330 |
| 3 unpurified | 1.24 | 0.0856 | 0.0496 |
| 3 purified | 0.0912 | 0.0274 | 0.0222 |

In these aspects, in which purification results in a lighter product, the change in color may also be assessed by such suitable methods as ASTM method D1209. In Table 2, samples were prepared by dissolving 675 mg of sample in 5 mL of phosphate buffer, and then UV absorbance measurements were taken.

In some aspects, the copolymer may contain constituents and/or byproducts which absorb at 315 nm but with an intensity such that the absorbance at 450 nm does not result in a visible yellow color. In these aspects, the purification method is suitable in removing the constituents such that absorbance at 315 nm is suitably reduced, from values in the range of 0.6 to 1.7 AU to values less than 0.1 AU.

Thus, in one aspect of the invention, a purification process is provided whereby the diblock copolymer is provided having a lighter color, e.g., a reduced yellowness, compared to the color of the starting diblock copolymer. The purification process entails precipitation of the copolymer and/or contact of a solution containing the copolymer with activated charcoal, both as described herein, in order to achieve a less intensely colored diblock copolymer. Purification of the diblock copolymer may be affected either before or after incorporating the additive into the composition, provided the additive and the copolymer will both precipitate. An example of such an additive is PEG 2000.

The compositions of the present invention may contain an additive. The additive may also be referred to as a solubilizing additive because one of its key functions is to assist in the solubilization of the components of a solvent-free composition when that solvent-free composition is combined with aqueous media. When present, the additive imparts advantageous properties to the composition. For example, the additive-containing compositions of the present invention are particularly advantageous in that they form micelles at an enhanced rate, have an enhanced ability to incorporate drug(s); and/or have advantageous physical characteristics, e.g., advantageous viscosity and/or melting point.

In one aspect of the present invention the additive is a polymer. The polymer may be hydrophilic, or the polymer may be hydrophobic. In one aspect, the polymer is hydrophilic. Optionally, the hydrophilic polymer has a molecular weight of 200-5,000. Optionally, the hydrophilic polymer is selected from poly(ethylene oxide) and the terminal $C_1$-$C_6$ alkyl ethers thereof. In a preferred embodiment, the additive polymer is MePEG. When the additive is MePEG, in one aspect, the MePEG has a molecular weight of 200 - 750 g/mol, while in another aspect the MePEG has a molecular weight of 550 - 2000 g/mol, while in yet another aspect the MePEG has a molecular weight of 750 - 5000 g/mol, where these molecular weight ranges are set froth in terms of number average molecular weight. Alternatively, the additive polymer may be hydrophobic. A suitable hydrophobic additive polymer is poly(DL-lactide). In one aspect, the hydrophobic additive polymer has a number average molecular weight of 288 to about 1,000.

In one aspect the present invention provides a composition that contains both diblock copolymer and additive polymer. For example, the present invention provides a composition comprising 1-15 parts diblock copolymer per each 1 part polymer. In another aspect, the present invention provides a composition comprising about 10 parts diblock copolymer per each 1 part polymer.

In addition to the polymer, or instead of using the polymer, the additive may be or include an organic solvent. Preferably, the organic solvent is biocompatible. The additive solvent should be biocompatible because it will be administered to the subject along with the drug and diblock copolymer. In one aspect, the solvent is N-methyl-2-pyrrolidone (NMP). In addition to NMP, PEG 200, propylene glycol, dimethylsulfoxide (DMSO) and analogs/homologues thereof are

exemplary biocompatible organic solvents. In one aspect, the solvent is selected from NMP and DMSO. Other solvents that might be suitable in the proper proportions include ethoxydiglycol and ethanol. The use of propylene glycol will typically require the attendant use of elevated temperatures in order to dissolve a desired amount of drug or block copolymer is the propylene glycol. In one aspect of the invention, the additive solvent is a combination of two or more biocompatible solvents, for example 2 solvents, or 3 solvents. In one aspect, the drug is soluble in the organic solvent or solvent mixture to a concentration of at least 1% w/v, *i.e.,* 1 weight part drug (in g) per 100 volume parts solvent (in mL). In other aspects, the drug is soluble in the organic solvent or solvent mixture to concentrations of at least 2% w/v, or 10% w/v, or 15% w/v, or 20% w/v, or 25% w/v, or 30% w/v, or 35% w/v, or 40% w/v. NMP is a preferred solvent in part because many drugs are very soluble in NMP. For instance, 535 mg of paclitaxel may be dissolved in 1 mL of NMP, while only 26 mg of paclitaxel may be dissolved in ethanol.

Drug solubility may be readily determined by adding solid drug to an aliquot of the solvent to be assessed. The drug-solvent mixture is allowed to equilibrate at 25±3°C for 8 to 24 hours. If the drug is completely dissolved resulting in a homogeneous clear mixture, additional drug is added and the process repeated until some solid drug remains undissolved over a period of at least 16 hours. After this point, the drug content in the liquid phase is determined by a suitable analytical technique such as chromatography, UV absorbance, or for acid or basic drugs, optionally titration. In some aspects, organic solvents are selected from those which dissolve the drug paclitaxel to an extent of at least 1% w/v. Solvents which exhibit this solubility property include 1-methyl-2-pyrrolidinone; ethoxtdiglycol, ethanol, propylene glycol, polyethylene glycol MW = 200, Tween 80.

In one aspect, the diblock copolymer is soluble in the organic solvent or solvent mixture to a concentration of at least 1% w/v, *i.e.,* 1 weight parts diblock copolymer per 100 volume parts solvent. In other aspects, the diblock copolymer is soluble in the organic solvent or solvent mixture to concentrations of at least 2% w/v, or 5% w/v, or 10% w/v, or 15% w/v, or 20% w/v, or 25% w/v, or 30% w/v, or 35% w/v, or 40% w/v. In another aspect, both the drug and the diblock copolymer are soluble in the organic solvent or solvent mixture to a concentration of at least 1% w/v. In other aspects, both the drug and the diblock copolymer are soluble in the organic solvent or solvent mixture to concentrations of at least 2% w/v, or 5% w/v, or 10% w/v, or 15% w/v, or 20% w/v, or 25% w/v, or 30% w/v, or 35% w/v, or 40% w/v.

The compositions and methods of the present invention include a drug. In particular, the inventive compositions and methods include a hydrophobic drug. Typically, hydrophobic drugs are very hard to incorporate into aqueous delivery vehicles because of their limited solubility in water. The present invention solves this problem by providing micelle-forming compositions, and micellar compositions, and methods of making and using same, that include the hydrophobic drug.

The term "hydrophobic drug" refers to drugs that are insoluble or sparingly or poorly soluble in water. As used herein, such drugs will have a solubility below 10 mg/ml, usually below 1 mg/ml, sometimes below 0.01 mg/ml, and sometimes below 0.001 mg/ml. Exemplary hydrophobic drugs include certain steroids, such as budesonide, testosterone, progesterone, estrogen, flunisolide, triamcinolone, beclomethasone, betamethasone; dexamethasone, fluticasone, methylprednisolone, prednisone, hydrocortisone, and the like; certain peptides, such as cyclosporin cyclic peptide, retinoids, such as all-cis retinoic acid, 13-trans retinoic acid, and other vitamin A and beta carotene derivatives; vitamins D, E, and K and water insoluble precursors and derivatives thereof; prostaglandins and leukotrienes and their activators and inhibitors including prostacyclin (epoprostanol), and prostaglandins; tetrahydrocannabinol; lung surfactant lipids; lipid soluble antioxidants; hydrophobic antibiotics and chemotherapeutic drugs such as amphotericin B and adriamycin and the like.

In one aspect, the hydrophobic drug is selected from the following classes of compounds: chemotherapeutic, antibiotic, antimicrotubule, anti-inflammatory, and antiproliferative compounds. In a preferred aspect, the hydrophobic drug is selected from paclitaxel, hydrophobic paclitaxel derivatives and hydrophobic paclitaxel analogs. In another preferred aspect, the hydrophobic drug is paclitaxel.

Within one preferred embodiment of the invention, the hydrophobic drug is paclitaxel, a compound currently recognized to disrupt mitosis (M-phase) by binding to tubulin to form abnormal mitotic spindles or an analogue or derivative thereof. Briefly, paclitaxel is a highly derivatized diterpenoid (Wani et al., J. Am. Chem. Soc. 93:2325, 1971). It may be obtained, for example, from the harvested and dried bark of *Taxus brevifolia* (Pacific Yew) and *Taxomyces Andreanae* and *Endophytic Fungus* of the Pacific Yew (Stierle et al., Science 60:214-216, 1993). "Paclitaxel" as used herein refers to hydrophobic formulations including paclitaxel, prodrugs, analogues and derivatives such as, for example, TAXOL®, TAXOTERE®, docetaxel, 10-desacetyl analogues of paclitaxel and 3'N-desbenzoyl-3'N-t-butoxy carbonyl analogues of paclitaxel) may be readily prepared utilizing techniques known to those skilled in the art *(see, e.g.,* Schiff et al., Nature 277:665-667, 1979; Long and Fairchild, Cancer Research 54:4355-4361, 1994; Ringel and Horwitz, J. Nat'l Cancer Inst. 83(4):288-291, 1991; Pazdur et al., Cancer Treat. Rev. 19(4):351-386, 1993; WO 94/07882; WO 94/07881; WO 94/07880; WO 94/07876; WO 93/23555; WO 93/10076; WO94/00156; WO 93/24476; EP 590267; WO 94/20089; U.S. Patent Nos. 5,294,637; 5,283,253; 5,279,949; 5,274,137; 5,202,448; 5,200,534; 5,229,529; 5,254,580; 5,412,092; 5,395,850; 5,380,751; 5,350,866; 4,857,653; 5,272,171; 5,411,984; 5,248,796; 5,248,796; 5,422,364; 5,300,638; 5,294,637; 5,362,831; 5,440,056; 4,814,470; 5,278,324; 5,352,805; 5,411,984; 5,059,699; 4,942,184; Tetrahedron Letters 35(52):

9709-9712, 1994; J. Med. Chem. 35:4230-4237, 1992; J. Med. Chem. 34:992-998, 1991; J. Natural Prod. 57(10): 1404-1410, 1994; J. Natural Prod. 57(11):1580-1583, 1994; J. Am. Chem. Soc. 110:6558-6560, 1988), or obtained from a variety of commercial sources, including for example, Sigma Chemical Co., St. Louis, Missouri (T7402 - from *Taxus brevifolia*).

Representative examples of paclitaxel derivatives and analogues include 7-deoxy-docetaxol, 7,8-cyclopropataxanes, N-substituted 2-azetidones, 6,7-epoxy paclitaxels, 6,7-modified paclitaxels, 10-desacetoxytaxol, 10-deacetyltaxol (from 10-deacetylbaccatin III), phosphonooxy and carbonate derivatives of taxol, taxol 2',7-di(sodium 1,2-benzenedicarboxylate, 10-desacetoxy-11,12-dihydrotaxol-10,12(18)-diene derivatives, 10-desacetoxytaxol, Protaxol (2'-and/or 7-O-ester derivatives ), (2'-and/or 7-O-carbonate derivatives), asymmetric synthesis of taxol side chain, fluoro taxols, 9-deoxotaxane, (13-acetyl-9-deoxobaccatine III, 9-deoxotaxol, 7-deoxy-9-deoxotaxol, 10-desacetoxy-7-deoxy-9-deoxotaxol, Derivatives containing hydrogen or acetyl group and a hydroxy and tertbutoxycarbonylamino, sulfonated 2'-acryloyltaxol and sulfonated 2'-O-acyl acid taxol derivatives, succinyltaxol, 2'-$\gamma$-aminobutyryltaxol formate, 2'-acetyl taxol, 7-acetyl taxol, 7-glycine carbamate taxol, 2'-OH-7-PEG(5000) carbamate taxol, 2'-benzoyl and 2',7-dibenzoyl taxol derivatives, other prodrugs (2'-acetyltaxol; 2',7-diacetyltaxol; 2'succinyltaxol; 2'-(beta-alanyl)-taxol); 2'gamma-aminobutyryltaxol formate; ethylene glycol derivatives of 2'-succinyltaxol; 2'-glutaryltaxol; 2'-(N,N-dimethylglycyl) taxol; 2'-(2-(N,N-dimethylamino)propionyl)taxol; 2'orthocarboxybenzoyl taxol; 2'aliphatic carboxylic acid derivatives of taxol, Prodrugs {2'(N,N-diethylaminopropionyl)taxol, 2'(N,N-dimethylglycyl)taxol, 7(N,N-dimethylglycyl)taxol, 2',7-di-(N,N-dimethylglycyl)taxol, 7(N,N-diethylaminopropionyl)taxol, 2',7-di(N,N-diethylaminopropionyl)taxol, 2'-(L-glycyl)taxol, 7-(L-glycyl)taxol, 2',7-di(L-glycyl)taxol, 2'-(L-alanyl)taxol, 7-(L-alanyl)taxol, 2',7-di(L-alanyl)taxol, 2'-(L-leucyl)taxol, 7-(L-leucyl)taxol, 2',7-di(L-leucyl)taxol, 2'-(L-isoleucyl)taxol, 7-(L-isoleucyl)taxol, 2',7-di(L-isoleucyl)taxol, 2'-(L-valyl)taxol, 7-(L-valyl)taxol, 2'7-di(L-valyl)taxol, 2'-(L-phenylalanyl)taxol, 7-(L-phenylalanyl)taxol, 2',7-di(L-phenylalanyl)taxol, 2'-(L-prolyl)taxol, 7-(L-prolyl)taxol, 2',7-di(L-prolyl)taxol, 2'-(L-lysyl)taxol, 7-(L-lysyl)taxol, 2',7-di(L-lysyl)taxol, 2'-(L-glutamyl)taxol, 7-(L-glutamyl)taxol, 2',7-di(L-glutamyl)taxol, 2'-(L-arginyl)taxol, 7-(L-arginyl)taxol, 2',7-di(L-arginyl)taxol}, Taxol analogs with modified phenylisoserine side chains, taxotere, (N-debenzoyl-N-tert-(butoxycaronyl)-10-deacetyltaxol, and taxanes (e.g., baccatin III, cephalomannine, 10-deacetylbaccatin III, brevifoliol, yunantaxusin and taxusin); and other taxane analogues and derivatives, including 14-beta-hydroxy-10 deacetybaccatin III, debenzoyl-2-acyl paclitaxel derivatives, benzoate paclitaxel derivatives, phosphonooxy and carbonate paclitaxel derivatives, sulfonated 2'-acryloyltaxol; sulfonated 2'-O-acyl acid paclitaxel derivatives, 18-site-substituted paclitaxel derivatives, chlorinated paclitaxel analogues, C4 methoxy ether paclitaxel derivatives, sulfenamide taxane derivatives, brominated paclitaxel analogues, Girard taxane derivatives, nitrophenyl paclitaxel, 10-deacetylated substituted paclitaxel derivatives, 14- beta -hydroxy-10 deacetylbaccatin III taxane derivatives, C7 taxane derivatives, C10 taxane derivatives, 2-debenzoyl-2-acyl taxane derivatives, 2-debenzoyl and -2-acyl paclitaxel derivatives, taxane and baccatin III analogs bearing new C2 and C4 functional groups, n-acyl paclitaxel analogues, 10-deacetylbaccatin III and 7-protected-10-deacetylbaccatin III derivatives from 10-deacetyl taxol A, 10-deacetyl taxol B, and 10-deacetyl taxol, benzoate derivatives of taxol, 2-aroyl-4-acyl paclitaxel analogues, orthro-ester paclitaxel analogues, 2-aroyl-4-acyl paclitaxel analogues and 1-deoxy paclitaxel and 1-deoxy paclitaxel analogues.

In one aspect, the hydrophobic drug is a taxane having the formula (1):

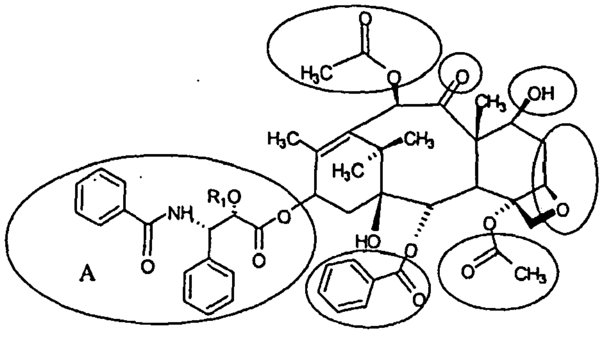

(1),

where the gray-highlighted portions may be substituted and the non-highlighted portion is the taxane core. A side-chain (labeled "A" in the diagram) is desirably present in order for the compound to have good activity as a cell cycle inhibitor. Examples of compounds having this structure include paclitaxel (Merck Index entry 7117), docetaxol (Taxotere, Merck Index entry 3458), and 3'-desphenyl-3'-(4-ntirophenyl)-N-debenzoyl-N-(t-butoxycarbonyl)-10-deacetyltaxol.

In one aspect, suitable taxanes such as paclitaxel and its hydrophobic analogs and derivatives are disclosed in Patent No. 5,440,056 as having the structure (2):

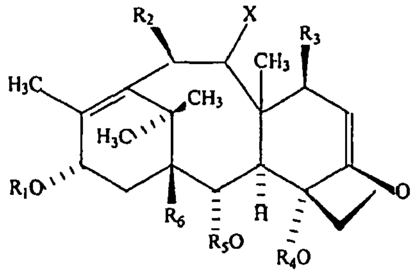

(2)

wherein X may be oxygen (paclitaxel), hydrogen (9-deoxy derivatives), thioacyl, or dihydroxyl precursors; $R_1$ is selected from paclitaxel or taxotere side chains or alkanoyl of the formula (3)

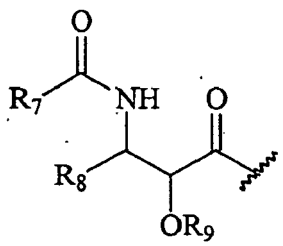

(3)

wherein $R_7$ is selected from hydrogen, alkyl, phenyl, alkoxy, amino, phenoxy (substituted or unsubstituted); $R_8$ is selected from hydrogen, alkyl, hydroxyalkyl, alkoxyalkyl, aminoalkyl, phenyl (substituted or unsubstituted), alpha or beta-naphthyl; and $R_9$ is selected from hydrogen, alkanoyl, substituted alkanoyl, and aminoalkanoyl; where substitutions refer to hydroxyl, sulfhydryl, allalkoxyl, carboxyl, halogen, thioalkoxyl, N,N-dimethylamino, alkylamino, dialkylamino, nitro, and $-OSO_3H$, and/or may refer to groups containing such substitutions; $R_2$ is selected from hydrogen or oxygen-containing groups, such as hydrogen, hydroxyl, alkoyl, alkanoyloxy, aminoalkanoyloxy, and peptidyalkanoyloxy; $R_3$ is selected from hydrogen or oxygen-containing groups, such as hydrogen, hydroxyl, alkoyl, alkanoyloxy, aminoalkanoyloxy, and peptidyalkanoyloxy, and may further be a silyl containing group or a sulphur containing group; $R_4$ is selected from acyl, alkyl, alkanoyl, aminoalkanoyl, peptidylalkanoyl and aroyl; $R_5$ is selected from acyl, alkyl, alkanoyl, aminoalkanoyl, peptidylalkanoyl and aroyl; $R_6$ is selected from hydrogen or oxygen-containing groups, such as hydrogen, hydroxyl alkoyl, alkanoyloxy, aminoalkanoyloxy, and peptidyalkanoyloxy.

In one aspect, the paclitaxel analogs and derivatives useful as a hydrophobic drug according to the present invention are disclosed in PCT International Patent Application No. WO 93/10076. As disclosed in this publication, the analog or derivative should have a side chain attached to the taxane nucleus at $C_{13}$, as shown in the structure below (formula 4), in order to confer antitumor activity to the taxane.

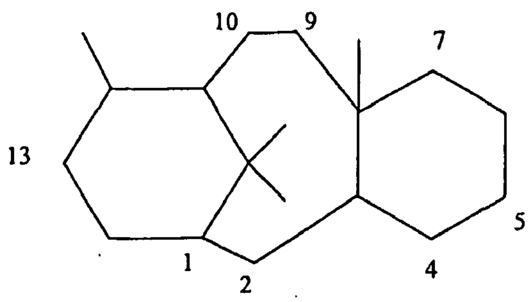

(4)

PCT International Publication No. WO 93/10076 discloses that the taxane nucleus may be substituted at any position with the exception of the existing methyl groups. The substitutions may include, for example, hydrogen, alkanoyloxy, alkenoyloxy, aryloyloxy. In addition, oxo groups may be attached to carbons labeled 2, 4, 9, 10. As well, an oxetane ring may be attached at carbons 4 and 5. As well, an oxirane ring may be attached to the carbon labeled 4.

In one aspect, the taxane-based hydrophobic drug useful in the present invention is disclosed in U.S. Patent 5,440,056, which discloses 9-deoxo taxanes. These are compounds lacking an oxo group at the carbon labeled 9 in the taxane structure shown above (formula C4). The taxane ring may be substituted at the carbons labeled 1, 7 and 10 (independently) with H, OH, O-R, or O-CO-R where R is an alkyl or an aminoalkyl. As well, it may be substituted at carbons labeled 2 and 4 (independently) with aryol, alkanoyl, aminoalkanoyl or alkyl groups. The side chain of formula (C3) may be substituted at $R_7$ and $R_8$ (independently) with phenyl rings, substituted phenyl rings, linear alkanes/alkenes, and groups containing H, O or N. $R_9$ may be substituted with H, or a substituted or unsubstituted alkanoyl group.

In another aspect, the taxane-based hydrophobic drug useful in the present invention is disclosed in U.S. Patent 6,107,332.

In a preferred aspect of the present invention, the inventive composition further comprising a buffering constituent. The buffering constituent is present so that, upon formation of an aqueous micellar solution, the solution has a physiological pH. In one aspect, the buffering constituent comprises a phosphate salt.

In a preferred aspect of the present invention, the inventive composition further comprising a neutralizing agent. The neutralizing agent is present so that acidic or basic groups present in the composition are converted into a salt form. Thus, upon addition to a water-containing composition of the invention, the neutralizing agent will form a salt with the acidic or basic components of the composition. Sufficient neutralizing agent is added to the composition to achieve a pH for the composition of at or near a physiological pH, *i.e.,* a pH of about 6.8 to 7.2. If the composition contains acidic material, so that the pH of the composition is below 7.0, then basic neutralizing agent may be added to the composition to increase the pH. If the composition contains basic materials, so that the pH of the composition is above 7.0, then acidic neutralizing agent may be added to the composition to decrease pH. Suitable basic neutralizing agents are strong bases, *e.g.,* sodium hydroxide, potassium hydroxide. Suitable acidic neutralizing agents are strong acids, e.g., hydrochloric acid. Upon formation of an aqueous micellar solution, the solution preferably has a physiological pH. The neutralizing agent present in the neutralized composition will be in the form of a salt, e.g., sodium ion and hydroxide ion, in the case where the neutralizing agent was sodium hydroxide.

In one aspect the present invention provides compositions that include diblock copolymer, additive selected from polymer and organic solvent, hydrophobic drug (particularly paclitaxel) and buffering constituent (particularly phosphate salt). The following compositions are exemplary compositions of the present invention, wherein paclitaxel is identified as a particular hydrophobic drug and phosphate salt is identified as a particular buffering constituent. Thus, in one aspect, the present invention provides a composition comprising 10-90 parts diblock copolymer, 10-70 parts additive selected from polymer and organic solvent, 1-15 parts paclitaxel and 1-20 parts phosphate salt. In another aspect, the present invention provides a composition comprising about 70 parts of diblock copolymer, about 7 parts polymer, about 8 parts paclitaxel and about 18 parts phosphate salt, the parts totaling 100. In another aspect, the present invention provides a composition comprising about 40 parts diblock copolymer, about 40 parts polymer, about 5 parts paclitaxel and about 11 parts phosphate salt, the parts totaling 100.

In another aspect, the present invention provides a composition comprising about 68 parts diblock copolymer having a weight ratio of methoxypolyethylene glycol block to poly(DL-lactide) block of about 60:40 and a molecular weight of about 3,300; about 7 parts of methoxypolyethylene glycol having a molecular weight of about 2,000; about 8 parts of paclitaxel; and about 18 parts phosphate salts, the parts in total equaling 100.

In another aspect, the present invention provides a composition comprising about 42 parts diblock copolymer having a weight ratio of methoxypolyethylene glycol block to poly(DL-lactide) block of about 60:40 and a molecular weight of about 3,300; about 42 parts of methoxypolyethylene glycol having a molecular weight of about 2,000; about 5 parts of paclitaxel; and about 11 parts phosphate salts, the parts in total equaling 100.

In another aspect, the present invention provides a composition comprising about 30 parts diblock copolymer having a weight ratio of methoxypolyethylene glycol block to poly(DL-lactide) block of about 60:40 and a molecular weight of about 3,300; about 60 parts of methoxypolyethylene glycol having a molecular weight of about 350; about 3 parts of paclitaxel; and about 8 parts phosphate salts, the parts in total equaling 100.

In another aspect, the present invention provides a composition comprising about 42 parts diblock copolymer having a weight ratio of methoxypolyethylene glycol block to poly(DL-lactide) block of about 60:40 and a molecular weight of about 3,300; about 42 parts of methoxypolyethylene glycol having a molecular weight of about 350; about 5 parts of paclitaxel; and about 11 parts phosphate salts, the parts in total equaling 100.

In another aspect, the present invention provides a composition comprising about 67 parts diblock copolymer having a weight ratio of methoxypolyethylene glycol block to poly(DL-lactide) block of about 60:40 and a molecular weight of about 3,300; about 8 parts of poly(DL-lactide) having a molecular weight of less than 3,000; about 8 parts of paclitaxel; and about 17 parts phosphate salts, the parts in total equaling 100.

In another aspect, the present invention provides a composition comprising about 71 parts diblock copolymer having a weight ratio of methoxypolyethylene glycol block to poly(DL-lactide) block of about 60:40 and a molecular

weight of about 3,300; about 3 parts of poly(DL-lactide) having a molecular weight of less than 3,000; about 8 parts of paclitaxel; and about 18 parts phosphate salts, the parts in total equaling 100.

In another aspect, the present invention provides a composition comprising about 56 parts diblock copolymer having a weight ratio of methoxypolyethylene glycol block to poly(DL-lactide) block of about 60:40 and a molecular weight of about 3,300; about 23 parts of N-methyl-2-pyrrolidone; about 6 parts of paclitaxel; and about 15 parts phosphate salts, the parts in total equaling 100.

The preparation of the non-aqueous compositions of the present invention can be accomplished in a number of ways. In one aspect, a solvent is selected that dissolves each of paclitaxel, diblock copolymer and additive. For example, diblock copolymer and polymer additive may be combined and heated to achieve a molten mixture. To this melt is then added a solution of paclitaxel in an organic solvent, preferably tetrahydrofuran. Other orders of combining the ingredients may be employed. Regardless of the order of combining the ingredients, in the end the three components are dissolved in the solvent to achieve a homogeneous composition. Sufficient solvent must be employed to achieve dissolution of the components. For example, about 200 grams of THF may be used to dissolve about 45 grams of paclitaxel, and this solution is combined with molten diblock copolymer and polymer additive to achieve a homogenous solution. In the solution, paclitaxel is about 9-10% by weight of the total mass of materials excluding solvent, *i.e.,* paclitaxel is about 9-10% by weight of the total weight of paclitaxel, diblock copolymer and additive.

The solvent is then removed. Removal of the solvent may be accomplished by exposing the composition to a reduced pressure, i.e., a vacuum, and/or an elevated temperature, i.e., a temperature greater than about 23°C, and/or to a stream of dry gas, e.g., nitrogen or argon. Under conditions of reduced pressure and/or elevated temperature and/or exposure to a streatm of dry gas, evaporation of the solvent is expedited. Preferably, both reduced pressure and elevated temperature are used to facilitate solvent removal. The drying process conditions of temperature and pressure and time may be varied to optimize the process for scale and equipment used. In small scale experiments, drying times range from 2 to 72 hours, temperatures range from ambient to 75°C, and gas environment ranges from forced air to reduced pressure to full vacuum. These conditions may be optimized for the scale of the reaction. The dry, or nearly dry residue resulting from this process can then be ground up to provide a homogeneous powder.

One shortcoming with this approach to preparing the non-aqueous compositions of the present invention is that paclitaxel tends to degrade when exposed to elevated temperature. The degradation can be mitigated by using relatively lower elevated temperature, *e.g.,* 35°C. However, as the elevated temperature is reduced, a corresponding increase in the time to achieve the same level of solvent evaporation is typically observed. Another shortcoming with this approach to preparing the non-aqueous compositions of the invention is that residual solvent typically remains in the product. This problem may, in part, be addressed by using as little solvent as possible in the preparation of the compositions. However, minimizing the solvent can only go part way toward solving the basic problem because some amount of solvent is typically necessary for the process to work successfully. For purposes of consistency and safety, the amount of residual solvent is desirably minimzied.

In order to avoid problems with residual solvent and paclitaxel degradation as discussed above, the present invention provides alternative methods of preparing the non-aqueous compositions. Thus, in one aspect, an organic solution of paclitaxel is prepared and then combined with a portion of the carrier, i.e., the diblock copolymer and the additive. Sufficient solvent is used to just dissolve all the components so that a homogeneous solution results. Slightly elevated temperature may be, and preferably is employed to achieve a completely homogeneous solution. Of course, the elevated temperature should be below the boiling point of the solvent and the degradation temperature of the drug. After a homogeneous solution has been achieved, the majority, if not all of the solvent is removed using reduced pressure and/or elevated temperature as discussed above. The residue, which will be molten at the elevated temperature, is then combined with the remaining carrier components while maintaining a liquid state. The homogeneous mixture is then cooled to room temperature whereupon it solidifies.

As illustrations of this process, the following options are provided, where any two or more options may be combined into a single process. 1. About 10% of the carrier matrix comprising diblock copolymer and MePEG 2000 in a weight ratio of 41.29:412.84 is combined with a THF solution of paclitaxel to provide a homogeneous solution from which the majority of the THF is subsequently removed; 2. The diblock copolymer is combined with the paclitaxel in a weight ratio of about 1:1 to 3:1; 3. The MePEG 2000 is combined with the paclitaxel is a weight ratio of about 1:1; 4. A 1:1 weight ratio of MePEG 2000 and diblock copolymer is prepared, and this composition is combined in a weight ratio of about 1:1 to 3:1 with paclitaxel.

Another method to prepare the water-free compositions of the present invention avoids the use of organic solvent altogether. According to this method, solid dibock copolymer and solid paclitaxel are combined and then mixed and/or milled to achieve a somewhat homogeneous mixture. This mixture is then melted to produce a substantially liquid composition. At this point the mixture may still contain some solid paclitaxel. The mixture is cooled and milled at low temperature, e.g., by contact with dry ice and the milled powder allowed to warm to room temperature. The milled powder is then heated to a molten state, typically achieved at 60-75°C. This process of melting and milling is repeated as needed until a homogeneous melt is obtained, i.e., a melt that is free of solid paclitaxel. Two cycles are typically sufficient to

achieve a homogeneous melt for the composition on a 5 gram scale. Additional heating/milling cycles may be employed as the amount of the components is increased. Also, as the scale increases, stirring speed may be adjusted as needed to increase the amount of paclitaxel that dissolves in the melt. Residual materials that resist melting after a number of heating/cooling cycles may be removed by filtration of the liquid, or by sieving of the powder. The residue powder may contain particles in the'micron size range.

In any of the compositions described above, unless water is specifically identified as being present in the composition, in one aspect the above-described compositions of the present invention have less than 5% moisture content. In another aspect, the above-described compositions of the present invention are in an anhydrous form. In a preferred aspect, the compositions that are anhydrous have been produced through lyophilization of a micellar solution.

The present invention provides compositions that, upon combination with water or other aqueous media, form an aqueous composition where the aqueous composition comprises micelles. Thus, in one aspect, the present invention provides a composition comprising (a) a biocompatible diblock copolymer (X-Y) having a hydrophilic block X and a hydrophobic block Y; (b) a water-soluble biocompatible organic solvent, (c) a hydrophobic drug; and (d) water; where the composition comprises micelles. In another aspect, the present invention provides a composition comprising (a) a biocompatible diblock copolymer (X-Y) having a hydrophilic block X, and a hydrophobic block Y; (b) a hydrophilic polymer; (c) a hydrophobic drug; and (d) water; where the composition comprises micelles. In one embodiment, the (b) hydrophilic polymer has a number average molecular weight that is less than the number average molecular weight of the (a) diblock copolymer.

In another aspect, the present invention provides a composition comprising (a) a biocompatible diblock copolymer (X-Y) having a hydrophilic block X, and a hydrophobic block Y; (b) a hydrophobic polymer; (c) a hydrophobic drug; and (d) water; where the composition comprises micelles. In one embodiment, the (b) hydrophobic polymer has a number average molecular weight that is less than the number average molecular weight of the diblock copolymer.

The compositions of the present invention that contain micelles may be readily prepared by adding aqueous media to an anhydrous or "low water content" composition, e.g., a composition having less than 5 wt% water, and then mixing the components together with some agitation. The aqueous media will necessarily include some water, and may be pure water, where pure water has less than 0.5 wt% dissolved solids. Pure water may be obtained by, e.g., distilling the water and/or subjecting the water to a deionization process. Both distillation and deionization of water is well known in the art, and many companies supply machines to efficiently purify water, see, e.g., Waters, Millipore, MA. The aqueous media, in addition to water, may contain dissolved salts, e.g., buffer such as phosphate buffer, or pH neutralizing agent such as a strong acid, *e.g.,* HCl, or a strong base, e.g., NaOH. The aqueous media may also, or alternatively, contain one or more pharmaceutically acceptable carriers, as identified below. The anhydrous or "low water content" composition of the present invention are particularly advantageous in that they form micelles at an enhanced rate, have an enhanced ability to incorporate drug(s); and/or have advantageous physical characteristics, e.g., advantageous viscosity and/or melting point.

Thus, in one particular aspect of the present invention, a method is provided for forming a drug delivery vehicle, comprising sequentially providing an aqueous or low water content composition as describe herein; and adding aqueous media to the composition to form a micelle-containing composition. In addition to aqueous media, any pharmaceutically acceptable carriers may be used to constitute the therapeutic composition from the precursor composition. Such carriers are well known in the pharmaceutical art, and are described, for example, in Remingtons Pharmaceutical Sciences, Mack Publishing Co. (A.R. Gennaro edit. 1985). For example, sterile saline and phosphate-buffered saline at physiological pH may be used. Preservatives, stabilizers, dyes and even flavoring agents may be provided in the composition. For example, sodium benzoate, sorbic acid and esters of *p*-hydroxybenzoic acid may be added as preservatives. Id. at 1449. In addition, antioxidants and suspending agents may be used. Id.

The compositions of the present invention that can be formed into a micelle-containing composition by the addition of aqueous media, are readily prepared simply by combining the individual ingredients and mixing them together. Where one or more of the components is a solid, it may be desirable to warm the admixture to an elevated temperature so that the admixture is a fluid, and then stir the fluid admixture to homogeneity prior to cooling.

In one particular aspect of the present invention, a method is provided for forming a composition that may be converted into a micelle-containing composition through the addition of aqueous media, comprising sequentially combining the diblock copolymer, additive and hydrophobic drug with an additional organic (processing) solvent; and then removing the organic (processing) solvent by evaporation or distillation. A suitable organic processing solvent is tetrahydrofuran, ethanol, acetonitrile, chloroform, and/or dichloromethane. In one embodiment, the admixture including the organic processing solvent is heated to between about 40-100°C to allow mixing and/or organic solvent removal.

As another aspect, the present invention provides a method of forming a composition which, upon combination with aqueous media forms a micelle, where the method comprises dissolving the hydrophobic drug in the additive and then adding the diblock copolymer to the additive. As an option, the diblock copolymer may also be dissolved in the additive. Again, as an option, the mixture may be heated to between 40 and 100°C to allow for dissolution of the component (s) in the additive.

A micellar solution according to the present invention is preferably clear. A suitable test for the clarity of a solution is as follows. An aliquot of test sample is placed in a Quartz cuvette having a path length of 1 cm. The cuvette is placed in a UV spectrophotometer set to measure absorbance at 450 nm. Prior to analysis of the sample, the UV spectrophotometer is blanked using a normal saline control. An absorbance value of not greater than 0.15 AU for the test sample indicates the presence of a clear micellar solution. If the solution is not clear, or contains some insoluble hydrogel, the solution may be filtered.

In one aspect, the compositions of the present invention are sterile. Many pharmaceuticals are manufactured to be sterile and this criterion is defined by the USP XXII <1211>. Sterilization in this embodiment may be accomplished by a number of means accepted in the industry and listed in the USP XXII <1211>, including gas sterilization, ionizing radiation or filtration. Sterilization may be maintained by what is termed asceptic processing, defined also in USP XXII <1211>. Acceptable gases used for gas sterilization include ethylene oxide. Acceptable radiation types used for ionizing radiation methods include gamma, for instance from a cobalt 60 source and electron beam. A typical dose of gamma radiation is 2.5 MRad. Filtration may be accomplished using a filter with suitable pore size, for example 0.22 $\mu$m and of a suitable material, for instance Teflon.

In another aspect, the compositions of the present invention are contained in a container that allows them to be used for their intended purpose, i.e., as a pharmaceutical composition. Properties of the container that are important are a volume of empty space to allow for the addition of a constitution medium, such as water or other aqueous medium, e.g., saline, acceptable light transmission characteristics in order to prevent light energy from damaging the composition in the container (refer to USP XXII <661>), an acceptable limit of extractables within the container material (refer to USP XXII), an acceptable barrier capacity for moisture (refer to USP XXII <671>) or oxygen. In the case of oxygen penetration, this may be controlled by including in the container, a positive pressure of an inert gas, such as high purity nitrogen, or a noble gas, such as argon. The term "USP" refers to U.S. Pharmacopeia (see www.usp.org, Rockville, MD).

Typical materials used to make containers for pharmaceuticals include USP Type I through III and Type NP glass (refer to USP XXII <661>), polyethylene, Teflon, silicone, and gray-butyl rubber. For parenterals, USP Types I to III glass and polyethylene are preferred.

In one aspect, the compositions of the present invention include one or more preservatives or bacteriostatic agents, present in an effective amount to preserve the composition and/or inhibit bacterial growth in the composition, for example, bismuth tribromophenate, methyl hydroxybenzoate, bacitracin, ethyl hydroxybenzoate, propyl hydroxybenzoate, erythromycin, chlorocresol, benzalkonium chlorides, and the like. Examples of the preservative include paraoxybenzoic acid esters, chlorobutanol, benzylalcohol, phenethyl alcohol, dehydroacetic acid, sorbic acid, etc. In one aspect, the compositions of the present invention include one or more bactericidal (also known as bacteriacidal) agents. In one aspect, the compositions of the present invention include one or more antioxidants, present in an effective amount. Examples of the antioxidant include sulfites and ascorbic acid. In one aspect, the compositions of the present invention include one or more coloring agents, also referred to as dyestuffs, which will be present in an effective amount to impart observable coloration to the composition. Examples of coloring agents include dyes suitable for food such as those known as F. D. & C. dyes and natural coloring agents such as grape skin extract, beet red powder, beta carotene, annato, carmine, turmeric, paprika, and so forth.

The present invention also provides a process of lyophilization, comprising lyophilization of the micelle-containing composition described above to create a lyophilized powder. In a preferred embodiment, the process further comprises reconstitution of the lyophilized powder with water or other aqueous media, such as benzyl alcohol-containing bacteriostatic water for injection, to create a reconstituted solution (Bacteriostatic Water for Injection, Abbott Laboratories, Abbott Park, Ill.).

In another aspect, the present invention provides a method of treating a disease in a mammal comprising the administration of an effective amount of a micelle-containing composition as described above, or a precursor thereof that will form micelles in the mammal, to the mammal. In another aspect, the present invention provides a method of preventing disease in a mammal comprising the administration of an effective amount of a micelle-containing composition as described above, or a precursor thereof that will form micelles in the mammal, to the mammal. In various aspects, the disease is selected from inflammatory conditions, neurological disorders, cancer, and benign hyperproliferative diseases. Thus, the disease may be arthritis, and/or the disease may be multiple sclerosis, and/or the disease may be Alzheimer's disease, and/or the disease may be psoriasis, and/or the disease may be cancer, and/or the disease may be stenosis or restenosis, and/or the disease may be benign hyperplasia, for example, benign hyperplasia induced by a foreign body, and/or the disease may be cardiovascular disease, and/or the disease may be Inflammatory Bowel Disease.

In one aspect, the composition is administered by a route selected from intravenous, intraarticular, intracutaneous, interstitial, subcutaneous, intramuscular injection, insertion into the rectum, oral, or implant into the body. Thus, the composition may be administered by intravenous delivery of an aqueous micelle solution. Alternatively, the composition may be administered by implanting a semi-solid composition in the body, where the semi-solid composition gradually delivers drug-containing micelles to the body.

In these therapeutic or prophylactic methods, a preferred hydrophobic drug being delivered by the method is paclitaxel or an analog or derivative thereof as described above.

In order to administer a composition of the present invention to a subject, in one method an aliquot of a micellar solution according to the present invention may be withdrawn using a syringe equipped with about a *ca.* 19 gauge needle. The aliquot is injected into an intravenous infusion bag and an additional quantity of 0.9%w/w saline solution is added to the infusion bag to yield a volume of 120 ml. The subject is then administered a therapeutically effective amount of the hydrophobic drug, from the intravenous infusion bag.

In general, the "therapeutically effective amount" of a hydrophobic drug according to the present invention will depend on the route of administration, the type of mammal being treated, and the physical characteristics of the specific mammal under consideration. These factors and their relationship to determining this amount are well known to skilled practitioners in the medical arts. This amount and the method of administration can be tailored to achieve optimal efficacy but will depend on such factors as weight, diet, concurrent medication and other factors which those skilled in the medical arts will recognize.

As another example of administering a composition of the present invention, liquid micelles that are substantially free of water may be loaded into a soft gelatin (or other water-soluble) capsule and administered by introduction into the gastrointestinal tract (orally or rectally) or by implantation into the body, such as insertion into a body cavity or by injection directly into a tissue. For example, 150 μg of liquid micelles (see, e.g., Example 5) may be loaded into a soft gelatin capsule and administered orally to a patient. The total dose given by this means would be equal to approximately 5 mg of paclitaxel. This dosage may be increased or decreased depending on the determination of the medical practitioner.

As another example, liquid micelles may be administered in a manner similar to that used for freeze-dried micellar paclitaxel. For instance, 7 g of liquid micelle may be contained in a sealed bottle, and then diluted with 25 ml saline, and an aliquot thereof further diluted prior to intravenous infusion. The liquid micelle may be injected directly into a patient, either into a cavity or directly into a tissue.

The following examples are offered by way of illustration, and not by way of limitation. In the Examples, paclitaxel was obtained from Hauser Chemical (Boulder, CO; www.hauser.com); DL-lactide was obtained from Purac America (Lincolnshire, IL); MePEG was obtained from Sigma (St. Louis, MO). The molecular weight and molecular weight distribution of a polymer or block copolymer may be determined using gel permeation chromatography (GPC) according to techniques well known in the art, where many manufacturers sell instruments for this purpose, *see, e.g.,* Waters, Milford, MA. Typically, the retention time(s) for a sample polymer, as determined by GPC, are compared to the retention time(s) of monodisperse polystyrene standards (which are commercially available from many suppliers, e.g., Aldrich Chemical, Waters, and Showa Denko, Japan are three representative suppliers), and this comparison provides a molecular weight measurement for the sample polymer. The average diameter of a micelle particle, and the size distribution of the particles, may be determined by light scattering techniques well known in the art. *See, e.g.,* K. Holmberg, ed., Handbook of Applied Surface and Colloid Chemistry, John Wiley & Sons, 2001. For instance, particle size may be determined by subjecting a micellar solution to dynamic light scattering (DLS) spectrometry, where a suitable spectrometer is available from many commercial suppliers, e.g., Lexel Laser Inc. Freemont, CA and Brookhaven Instruments Co., Holtsville, NY. The spectrometer can be set at a wavelength of about 500 nm and a temperature of about 25°C in making the measurements. A suitable detector for the scattered light is a photomultiplier, where photomultipliers are likewise available from many commercial suppliers, e.g., Brookhaven Instruments Co., Holtsville, NY.

## EXAMPLES

### EXAMPLE 1

### PREPARATION OF 60:40 MEPEG:POLY(DL-LACTIDE) DIBLOCK COPOLYMER

A 60:40 MePEG:poly(DL-lactide) diblock copolymer was prepared by combining 60 g of DL-lactide and 40 g of MePEG (MW = 2,000 g/mol) in a round bottom glass flask containing a TEFLON™-coated stir bar. The mixture was heated to 140°C with stirring in a temperature controlled mineral oil bath until the components melted to form a homogeneous liquid. 0.1 g of stannous 2-ethyl hexanoate was added to the molten mixture and the reaction was continued for 6 hours at 140°C with continuous stirring. The reaction was terminated by cooling the product to ambient temperature. The product, 60:40 MePEG:poly(DL-lactide) diblock copolymer was stored in sealed containers at 2-8°C until use.

EXAMPLE 2

PRECIPITATION OF 60:40 MEPEG:POLY(DL-LACTIDE) DIBLOCK COPOLYMER FROM ISOPROPANOL

A 60:40 MePEG:poly(DL-lactide) diblock copolymer was prepared according to the method in Example 1. The copolymer (47 g) was dissolved in isopropanol to make a 5%w/v solution. The mixture was heated to 50°C for 2 hours with shaking every 30 minutes. The result was a clear solution. The solution was cooled to 2°C over a 16 hour period in order to precipitate the copolymer. The mixture was centrifuged for 20 minutes at 3000 rpm to pelletize the copolymer and the supernatant was removed and replaced with fresh isopropanol. The heating (to dissolve) and cooling (to precipitate) cycle was repeated twice more. After the final precipitation step, the copolymer pellet was transferred to a vacuum oven cooled to -10°C and dried for 5 hours. The oven was heated to ambient temperature and the vacuum maintained for a further 10 hours. The result was copolymer recovered as a white powder.

At various times in drying, the copolymer was analyzed by head space gas chromatography to measure the amount of isopropanol remaining in the matrix. The analysis was performed using a Supelcowax-10 GC column (30 m x 530 μm x 1.00 μm nominal), an injection port temperature of 140°C, detector temperature of 260°C and column temperature of 50°C for 9 minutes after injection, increasing thereafter to 100°C at 12°C/min. Typical values of isopropanol remaining were as follows:

| Drying time (h) | Lower Range (%/w/w) | Upper Range (%w/w) |
|---|---|---|
| 42 | 0.05 | 1.32 |
| 104 | 0.02 | 0.04 |

Higher values were observed in a batch made which resulted in a coarser powdered product. In this batch the amounts of isopropanol remaining were as follows:

| Drying time (h) | Lower Range (%/w/w) | Upper Range (%w/w) |
|---|---|---|
| 42 | 5.87 | 6.74 |
| 104 | 1.57 | 6.03 |

In order to test an alternate drying system, prior to drying the copolymer by reduced pressure, small aliquots of approximately 3 grams were removed and placed at 50-55°C with heated air forced over the samples. At intervals of 24 and 48 hours, these small samples were removed at analyzed in the same manner by GC. In these samples, no isopropanol was detected. The limit of detection of the assay is less than 0.01% w/w.

An alternative GC method may be found for isopropanol in USP XXIV <467>.

Accordingly, in various aspects the present invention provides diblock copolymer having isopropanol contamination of less than 10% w/w, less than 5% w/w/, less than 1% w/w, less than 0.1% w/w/, less than 0.01% w/w. A preferred diblock copolymer having minimal isopropanol contamination is a polyester-polyether diblock coplymer, and a further preferred diblock copolymer is a poly(ethylene glycol)-poly(lactide) copolymer. Drying techniques as described herein may also be used to remove other solvent contamination, so as to provide diblock copolymer having solvent contamination of less than 10% w/w, less than 5% w/w/, less than 1% w/w, less than 0.1% w/w/, and less than 0.01% w/w, where an exemplary contaminating solvents besides isopropanol is tetrahydrofuran.

EXAMPLE 3

PREPARATION OF FREEZE DRIED MICELLAR PACLITAXEL

A solid composition capable of forming micelles upon constitution with an aqueous medium was prepared as follows. 3.5 g of MePEG (MW = 2,000 g/mol) was combined with 0.175 g of paclitaxel and heated in a mineral oil bath to 100°C. The mixture was stirred until the paclitaxel dissolved in the molten MePEG. After a clear liquid was formed, 1.75 g of 60:40 MePEG:poly(DL-lactide) diblock copolymer (see Example 1) was added and allowed to melt. The mixture was further stirred to ensure a homogeneous mixture. The composition was cooled to ambient temperature and dissolved in 8.84 ml of a phosphate buffered saline. Dissolution was accomplished by stirring the mixture until a clear solution resulted. Using an automatic pipette, 0.6 ml of the solution was transferred to a 10 ml glass bottle and the material freeze dried by cooling to -34°C, heating to -20°C while reducing pressure to less than 0.2 mm Hg, holding for 24 hours, heating

to 30°C while maintaining low pressure, followed by holding for a further 12 hours. The result was a freeze dried matrix that could be constituted to form a clear micellar solution.

EXAMPLE 4

PREPARATION OF FREEZE DRIED MICELLAR PACLITAXEL

A solid composition capable of forming micelles upon constitution with an aqueous medium was prepared as follows. 41.29 g of MePEG (MW = 2,000 g/mol) was combined with 412.84 g of 60:40 MePEG:poly(DL-lactide) diblock copolymer *(see, e.g.,* Example 1) in a stainless steel beaker, heated to 75°C in a mineral oil bath and stirred by an overhead stirring blade. Once a clear liquid was obtained, the mixture was cooled to 55°C. To the mixture was added a 200 ml solution of 45.87 g paclitaxel in tetrahydrofuran. The solvent was added at approximately 40 ml/min and the mixture stirred for 4 hours at 55°C. After mixing for this time, the liquid composition was transferred to a stainless steel pan and placed in a forced air oven at 50°C for about 48 hours to remove residual solvent. The composition was then cooled to ambient temperature and was allowed to solidify to form paclitaxel-polymer matrix.

A phosphate buffer was prepared by combining 237.8 g of dibasic sodium phosphate heptahydrate, 15.18 g of monobasic sodium phosphate monohydrate in 1600 ml of water. To the phosphate buffer, 327 g of the paclitaxel-polymer matrix was added and stirred for 2 hours to dissolve the solids. After a clear solution was achieved, the volume was adjusted to 2000 ml with additional water. Vials were filled with 15 ml aliquots of this solution and freeze dried by cooling to -34°C, holding for 5 hours, heating to -16°C while reducing pressure to less than 0.2 mm Hg, holding for 68 hours, heating to 30°C while maintaining low pressure, followed by holding for a further 20 hours. The result was a freeze dried matrix that could constituted to form a clear micellar solution.

EXAMPLE 5

PREPARATION OF SEMI-SOLID MICELLAR PACLITAXEL

A 2.24 g quantity of 60:40 MePEG:poly(DL-lactide) diblock copolymer was combined with 0.224 g of paclitaxel and the mixture heated to 50°C in a petri dish heated in a hot water bath. The diblock copolymer was allowed to melt and the paclitaxel was mixed into the liquid to form a slurry. The mixture was then cooled to ambient temperature. A 0.110 g aliquot of this material was transferred to a glass vial and 0.200 g of MePEG (MW = 350 g/mol) added. This mixture was again heated to 50°C in the hot water bath and stirred until a clear liquid was achieved. This material was cooled to ambient temperature to form a liquid containing some precipitated components. The semi-solid formulation was. formed into micelles by the addition of 5.4 ml of a phosphate buffered saline solution. The result was a clear micellar solution.

EXAMPLE 6

COMPOSITIONS USEFUL IN PREPARING PACLITAXEL-CONTAINING MICELLE-FORMING LIQUIDS

Liquid micellar paclitaxel may be prepared by combining 1-methyl-2-pyrrolidinone (NMP) and a diblock copolymer to produce a micelle forming matrix, for example, as follows: NMP and 60:40 MePEG:poly(DL-lactide) diblock copolymer *(see, e.g.,* Example 1) were combined in ratios of 10:1, 5:1, 2.5:1, and 1:1 NMP:diblock copolymer and the mixtures heated to 60°C in an oven until a clear liquid was formed. Each mixture was stirred to ensure homogeneity. The mixtures were allowed to cool to ambient temperature and were observed after 2.5 hours. All ratios except the 1:1 ratio showed no evidence of solidification after 2.5 hours. Another solvent with similar properties is dimethylsulfoxide. Other solvents may also be suitable but not at all ratios. For instance, diblock copolymer from Example 1 may be dissolved in ethanol or ethoxydiglycol at lower concentrations such as 5% w/v. Still other solvents may have suitable properties in only limited temperature ranges, for instance PEG 200 will dissolve diblock copolymer from Example 1 at a concentration of 5% w/v at temperatures close to 40°C.

Compositions which are clear or hazy and do not exhibit the presence of a solid phase may be suitable liquid compositions providing they also pass the test of forming a micellar solution with a characteristic critical micelle concentration and a complete solution in an aqueous medium, defined as having a UV absorbance of the resulting aqueous solution of less than 0.15 AU at 450 nm.

EXAMPLE 7

DETERMINATION OF COMPLETENESS OF A MICELLAR SOLUTION

To a composition containing 2.025 g diblock copolymer (60:40 MePEG 2000:poly(DL-lactide), MW about 3,300 g/mol), 0.2025 g MePEG 2000, 0.225 g paclitaxel, 0.894 g dibasic sodium phosphate dihydrate and 0.051 g monobasic sodium phosphate, monohydrate in a lyophilized matrix was added 25 ml of 0.9% sodium chloride in water. The mixture was shaken at room temperature until the non-aqueous matrix dissolved in the water.

This aqueous composition was analyzed for its UV absorbance in a Quartz cuvette having a path length of 1 cm using a UV spectrophotometer set to measure absorbance at 450 nm. Prior to analysis the UV spectrophotometer was blanked using a normal saline control. An absorbance value of not greater than 0.15 AU was observed for the test sample, indicating the presence of a clear micellar solution.

EXAMPLE 8

CONSTITUTION OF FREEZE-DRIED MICELLAR PACLITAXEL

Freeze-dried micellar paclitaxel was constituted by adding 25 ml of sterile 0.9%w/w saline solution to the solid composition by injection into the sealed container using a syringe equipped with about a 19 gauge needle. The solid matrix contains, in this Example, 225 mg paclitaxel, 2.025 g of 60:40 MePEG:poly(DL-lactide) diblock copolymer, 0.2025 g MePEG (MW = 2,000 g/mol), 892 mg of dibasic sodium phosphate heptahydrate and 50.1 mg of monobasic sodium phosphate monohydrate. After the saline solution was added, the syringe was withdrawn from the container and the components were mixed by shaking for about 2 minutes. The vial was then left to stand for up to 10 minutes to allow any bubbles to dissipate from the solution. The product was visibly clear.

EXAMPLE 9

ADMINISTRATION OF MICELLAR PACLITAXEL

Freeze-dried micellar paclitaxel may be constituted as described in Example 8 and an aliquot of the micellar solution withdrawn using a syringe equipped with about a 19 gauge needle. The aliquot is injected into an intravenous infusion bag and an additional quantity of 0.9%w/w saline solution is added to the infusion bag to yield a volume of 120 ml. The patient is administered 100 ml of the solution in the bag. In this example an aliquot of 14.5 ml withdrawn from the product vial will result in about a 100 mg dose administered to the patient.

EXAMPLE 10

ADMINISTRATION OF MICELLAR PACLITAXEL

Liquid micelles that are substantially free of water may be loaded into a soft gelatin (or other water-soluble) capsule and administered by introduction into the gastrointestinal tract (orally or rectally) or by implantation into the body, such as insertion into a body cavity or by injection directly into a tissue. For example, 150 μg of liquid micelles (see, e.g., Example 6) may be loaded into a soft gelatin capsule and administered orally to a patient. The total dose given by this means would be equal to approximately 5 mg of paclitaxel.

EXAMPLE 11

ADMINISTRATION OF MICELLAR PACLITAXEL

Liquid micelles may be administered in a manner similar to that used for freeze-dried micellar paclitaxel. Thus, 7 g of liquid micelle may be contained in a sealed bottle. The liquid is diluted with 25 ml saline and an aliquot further diluted prior to intravenous infusion. The liquid micelle may be injected directly into a patient, either into a cavity or directly into a tissue.

From the foregoing, it will be appreciated that, although specific embodiments of the invention have been described herein for purposes of illustration, various modifications may be made without deviating from the spirit and scope of the invention.

Aspects of the present disclosure will be set out below in the following numbered clauses:

1. A composition comprising:

(a) a micelle-forming biocompatible diblock copolymer (X-Y) having a hydrophilic block X comprising residues of monomer x, and a hydrophobic block Y comprising residues of monomer y;
(b) an additive comprising at least one of a polymer or a water soluble, biocompatible, organic solvent; and
(c) a hydrophobic drug.

2. A composition comprising:

(a) a micelle-forming biocompatible diblock copolymer (X-Y) having a hydrophilic block X comprising residues of monomer x, and a hydrophobic block Y comprising residues of monomer y;
(b) an additive comprising at least one of a polymer and an organic solvent,

i) the solvent being water-soluble and biocompatible;
ii) the polymer comprising monomer residues x and/or y; and

(c) a hydrophobic drug;

with the proviso that the composition forms a micellar solution in water.

3. A composition comprising:

(a) a biocompatible diblock copolymer (X-Y) having a block X comprising residues of monomer x, and a block Y comprising residues of monomer y, the block X being more hydrophilic than the block Y;
(b) a biocompatible water-soluble additive comprising at least one of a polymer and an organic solvent; and
(c) a hydrophobic drug;

with the proviso that the composition forms a micellar solution in aqueous media.

4. The composition of clauses 1-3 wherein the block X comprises residues of one or more monomers selected from (meth)acrylic acid, vinylpyrrolidone, saccharide, and amino acid.

5. The composition of clauses 1-3 wherein the block X comprises residues of alkylene oxide.

6. The composition of clauses 1-3 wherein X is selected from poly(acrylic acid), poly(vinyl pyrrolidone), poly(saccharide), poly(amino acid).

7. The composition of clauses 1-3 wherein X comprises poly(alkylene oxide).

8. The composition of clause 7 wherein the poly(alkylene oxide) is selected from poly(ethylene oxide) and terminal $C_1$-$C_6$ alkyl ethers of poly(ethylene oxide).

9. The composition of clauses 1-3 wherein the block Y comprises residues of monomers selected from methacrylic acid, esters of methacrylic acid, esters of acrylic acid, and vinyl acetate.

10. The composition of clauses 1-3 wherein the block Y comprises residues of monomers selected from lactic acid and reactive equivalents thereof, glycolic acid and reactive equivalents thereof, and caprylic acid and reactive equivalents thereof.

11. The composition of clause 10 wherein the block Y is poly-DL-lactide-co-glycolide.

12. The composition of clause 10 wherein the block Y is poly-DL-lactide.

13. The composition of clauses 1-3 wherein the block Y is selected from polylactide, polyglycolide, polycaprolactone, hydrophobic polypeptides, hydrophobic polycarbonates, poly(vinyl acetate) and copolymers thereof.

14. The composition of clauses 1-3 wherein X comprises residues of monomers selected from alkylene oxide, acrylic acid, vinyl pyrrolidone, saccharide, and amino acid, while Y comprises residues of monomers selected from lactide

or reactive equivalents thereof, glycolide or reactive equivalents thereof, caprolactone or reactive equivalents thereof, hydrophobic amino acid, carbonate, and vinyl acetate.

15. The composition of clauses 14 wherein X comprises residues of monomers selected from alkylene oxide while Y comprises residues of monomers selected from lactide or reactive equivalents thereof and glycolide or reactive equivalents thereof.

16. The composition of clause 14 wherein X comprises residues of ethylene oxide while Y comprises residues of lactide.

17. The composition of clause 14 wherein X is MePEG and Y is poly(DL-lactide).

18. The composition of clauses 1-17 wherein 100 parts of diblock copolymer comprises 30-90 parts hydrophilic polymer X and 60-10 parts hydrophobic polymer Y.

19. The composition of clause 18 wherein 100 parts of diblock copolymer comprise 40-80 parts hydrophilic polymer X and 60-20 parts hydrophobic polymer Y.

20. The composition of clause 18 wherein 100 parts of diblock copolymer comprise 50-70 parts hydrophilic polymer X and 50-30 parts hydrophobic polymer Y.

21. The composition of clause 18 wherein 100 parts of diblock copolymer comprise about 60 parts hydrophilic polymer X and about 40 parts hydrophobic polymer Y.

22. The composition of clauses 1-21 wherein the diblock copolymer has a number average molecular weight of about 1,000 to about 10,000 g/mol.

23. The composition of clause 22 wherein the diblock copolymer has a number average molecular weight of about 2,000 to about 5,000 g/mol.

24. The composition of clause 22 wherein the diblock copolymer has a number average molecular weight of about 2,500 to about 3,500 g/mol.

25. The composition of any one of clauses 1-3 wherein the additive comprises a polymer.

26. The composition of clause 25 wherein the polymer is hydrophilic.

27. The composition of clause 25 wherein the polymer has a molecular weight of 200-5,000.

28. The composition of clause 25 wherein the polymer is selected from poly(ethylene oxide) and the terminal $C_1$-$C_6$alkyl ethers thereof.

29. The composition of any one of clauses 25 and 28 wherein the polymer is MePEG.

30. The composition of clause 29 wherein the MePEG has a molecular weight of 200 - 750 g/mol

31. The composition of clause 29 wherein the MePEG has a molecular weight of 550 - 2000 g/mol

32. The composition of clause 29 wherein the MePEG has a molecular weight of 750 - 5000 g/mol.

33. The composition of clause 25 wherein the polymer is hydrophobic.

34. The composition of clause 33 wherein the polymer is poly(DL-lactide).

35. The composition of clause 33 wherein the number average molecular weight of the polymer is 288 to about 1,000.

36. The composition of clauses 1-35 comprising 1-15 parts diblock copolymer per each 1 part polymer.

37. The composition of clause 36 comprising about 10 parts diblock copolymer per each 1 part polymer.

38. The composition of clauses 1-3 wherein the additive comprises an organic solvent.

39. The composition of clause 38 wherein the solvent is N-methyl-2-pyrrolidone (NMP).

40. The composition of clause 39 wherein copolymer is dissolved in the NMP at a concentration of 5 grams diblock copolymer in 100 mL NMP.

41. The composition of clause 39 wherein the copolymer is dissolved in the NMP at a concentration of 10 grams diblock copolymer in 100 mL NMP.

42. The composition of clauses 1-3 wherein the hydrophobic drug is selected from the following classes of compounds: chemotherapeutic, antibiotic, antimicrotubule, anti-inflammatory, and antiproliferative.

43. The composition of clauses 1-3 wherein the drug is selected from paclitaxel, paclitaxel derivatives and paclitaxel analogs.

44. The composition of clauses 1-3 wherein the drug is paclitaxel.

45. The composition of clauses 1-3 further comprising a buffering constituent.

46. The composition of clause 45 wherein the buffering constituent comprises a phosphate salt.

47. The composition of clauses 1-3 further comprising the dissolved form of a neutralizing agent.

48. The composition of clause 47 wherein the neutralizing agent is an acid.

49. The composition of clause 48 wherein the acid is hydrochloric acid.

50. The composition of clause 47 wherein the neutralizing agent is a base.

51. The composition of clause 50 wherein the base is sodium hydroxide.

52. The composition of clauses 1-3 comprising 10-90 parts diblock copolymer, 10-70 parts additive selected from polymer and organic solvent, 1-15 parts paclitaxel and 1-20 parts phosphate salt.

53. The composition of clauses1-3 comprising about 70 parts of diblock copolymer, about 7 parts polymer, about 8 parts paclitaxel and about 18 parts phosphate salt, the parts totaling 100.

54. The composition of clauses 1-3 comprising about 40 parts diblock copolymer, about 40 parts polymer, about 5 parts paclitaxel and about 11 parts phosphate salt, the parts totaling 100.

55. The composition of clauses 1-3 comprising
about 68 parts diblock copolymer having a weight ratio of methoxypolyethylene glycol block to poly(DL-lactide) block of about 60:40 and a molecular weight of about 3,300;
about 7 parts of methoxypolyethylene glycol having a molecular weight of about 2,000;
about 8 parts of paclitaxel; and
about 18 parts phosphate salts, the parts in total equaling 100.

56. The composition of clauses 1-3 comprising
about 42 parts diblock copolymer having a weight ratio of methoxypolyethylene glycol block to poly(DL-lactide) block of about 60:40 and a molecular weight of about 3,300;
about 42 parts of methoxypolyethylene glycol having a molecular weight of about 2,000;
about 5 parts of paclitaxel; and
about 11 parts phosphate salts, the parts in total equaling 100.

57. The composition of clauses 1-3 comprising

about 30 parts diblock copolymer having a weight ratio of methoxypolyethylene glycol block to poly(DL-lactide) block of about 60:40 and a molecular weight of about 3,300;
about 60 parts of methoxypolyethylene glycol having a molecular weight of about 350;
about 3 parts of paclitaxel; and
about 8 parts phosphate salts, the parts in total equaling 100.

58. The composition of clauses 1-3 comprising
about 42 parts diblock copolymer having a weight ratio of methoxypolyethylene glycol block to poly(DL-lactide) block of about 60:40 and a molecular weight of about 3,300;
about 42 parts of methoxypolyethylene glycol having a molecular weight of about 350;
about 5 parts of paclitaxel; and
about 11 parts phosphate salts, the parts in total equaling 100.

59. The composition of clauses 1-3 comprising
about 67 parts diblock copolymer having a weight ratio of methoxypolyethylene glycol block to poly(DL-lactide) block of about 60:40 and a molecular weight of about 3,300;
about 8 parts of poly(DL-lactide) having a molecular weight of less than 3,000;
about 8 parts of paclitaxel; and
about 17 parts phosphate salts, the parts in total equaling 100.

60. The composition of clauses 1-3 comprising
about 71 parts diblock copolymer having a weight ratio of methoxypolyethylene glycol block to poly(DL-lactide) block of about 60:40 and a molecular weight of about 3,300;
about 3 parts of poly(DL-lactide) having a molecular weight of less than 3,000;
about 8 parts of paclitaxel; and
about 18 parts phosphate salts, the parts in total equaling 100.

61. The composition of clauses 1-3 comprising
about 56 parts diblock copolymer having a weight ratio of methoxypolyethylene glycol block to poly(DL-lactide) block of about 60:40 and a molecular weight of about 3,300;
about 23 parts ofN-methyl-2-pyrrotidone;
about 6 parts of paclitaxel; and
about 15 parts phosphate salts, the parts in total equaling 100.

62. The composition of clauses 1-61 having less than 5% moisture content.

63. The composition of clauses 1-61 having less than 5% moisture content and being sterile.

64. The composition of clause 62 being in an anhydrous form.

65. The composition of clause 63 being in anhydrous form.

66. The composition of clause 62 being produced through lyophilization of a micellar solution.

67. The composition of clause 63 being produced through lyophilization of a micellar solution.

68. The composition of clauses 63, 65, or 67 that is packaged within a container that maintains the sterility of the composition.

69. The composition of clause 68 wherein the composition is packaged within packaging comprising a glass container with a sealed closure.

70. The composition of clause 68 wherein the composition is packaged within packaging comprising a plastic container with a sealed closure.

71. The composition of clauses 68, 69, or 70 wherein the packaging further comprises a sufficient volume of empty space to allow for the addition of water in a sufficient amount to produce a micelle-containing composition.

72. The composition of clauses 68, 69, or 70 wherein the packaging is substantially opaque to UV or visible light.

73. The composition of clauses 68, 69, or 70 wherein the packaging is substantially impervious to oxygen from air.

74. The composition of clauses 62-67 further comprising a bacteriacidal or bacteriostatic compound.

75. The composition of clauses 62-67 further comprising an antioxidant.

76. The composition of clauses 62-67 further comprising a coloring agent.

77. A method of producing the composition according to clauses 63, 65 or 67 by treating the composition according to a sterilization process selected from sterile filtration, sterilization with ethylene oxide, and sterilization with ionizing radiation.

78. The composition of clauses 1-61 further comprising water to form an aqueous composition, the aqueous composition comprising micelles.

79. A composition comprising

(a) a biocompatible diblock copolymer (X-Y) having a hydrophilic block X and a hydrophobic block Y;
(b) a water-soluble biocompatible organic solvent,
(c) a hydrophobic drug; and
(d) water;

wherein the composition comprises micelles.

80. A composition comprising

(a) a biocompatible diblock copolymer (X-Y) having a hydrophilic block X, and a hydrophobic block Y;
(b) a hydrophilic polymer;
(c) a hydrophobic drug; and
(d) water;

wherein the composition comprises micelles.

81. A composition comprising

(a) a biocompatible diblock copolymer (X-Y) having a hydrophilic block X, and a hydrophobic block Y;
(b) a hydrophobic polymer;
(c) a hydrophobic drug; and
(d) water;

wherein the composition comprises micelles.

82. A method for forming a drug delivery vehicle, comprising sequentially

(a) providing a composition according to any of clauses 1-67; and
(b) adding water to the composition of part (a) to form a micelle-containing composition.

83. A method of forming a composition of any one of clauses 1-3 comprising sequentially
combining the diblock copolymer, additive and hydrophobic drug with an additional organic (processing) solvent; and removing the organic (processing) solvent by evaporation or distillation.

84. A method according to clause 83 wherein the organic (processing) solvent comprises a solvent selected from tetrahydrofuran, ethanol, acetonitrile, chloroform, and dichloromethane.

85. A method according to clause 83 wherein the method further comprises heating the mixture to between 40-100°C to allow mixing and/or organic solvent removal.

86. A method of forming a composition of any one of clauses 1-3 comprising dissolving the hydrophobic drug in the additive and then adding the diblock copolymer to the additive.

87. A method according to clause 86 wherein the diblock copolymer is also dissolved in the additive.

88. A method according to either of clauses 83 or 86 wherein the mixture is heated to between 40 and 100°C to allow for dissolution of the component(s) in the additive.

89. A method of forming a composition of any one of clause 1-3 comprising

(a) combining the hydrophobic drug, the biocompatible diblock copolymer (X-Y) having a hydrophilic block X and a hydrophobic block Y, and the additive to form a homogeneous solution;
(b) removing some or all of the organic solvent from the solution of (a) to provide a low-solvent mixture; and
(c) combining additional biocompatible diblock copolymer (X-Y) having a hydrophilic block X comprising residues of monomer x and a hydrophobic block Y comprising residues of monomer y, with the low-solvent mixture of (b).

90. The method of clause 89 wherein the dibock copolymer is a polyether-polyester.

91. A method of forming a composition of any one of clauses 1-3 comprising

(a) combining the hydrophobic drug, the biocompatible diblock copolymer (X-Y) having a hydrophilic block X and a hydrophobic block Y, and the additive to form a homogeneous solution;
(b) removing some or all of the organic solvent from the solution of (a) to provide a low-solvent mixture; and
(c) combining additional additive organic solvent with the low-solvent mixture of (b).

92. The method of clause 91 wherein the additive organic solvent is tetrahydrofuran.

93. A method of forming a composition of any one of clauses 1-3 comprising

(a) combining the hydrophobic drug, the biocompatible diblock copolymer (X-Y) having a hydrophilic block X and a hydrophobic block Y, and the additive to form a homogeneous solution;
(b) removing some or all of the organic solvent from the solution of (a) to provide a low-solvent mixture; and
(c) combining additional additive polymer with the low-solvent mixture of (b).

94. The method of clause 93 wherein the additive polymer is a polyether.

95. The method of clauses 89, 91 or 93 wherein the drug is paclitaxel.

96. A composition prepared by the method of clauses 89, 91 or 93.

97. A composition formed by

(a) combining organic solvent, hydrophobic drug, biocompatible diblock copolymer (X-Y) having a hydrophilic block X comprising residues of monomer x and a hydrophobic block Y comprising residues of monomer y, and an additive selected from a polymer and a water soluble biocompatible organic liquid, to form a homogeneous solution; and
(b) removing some or all of the organic solvent from the solution of (a) to provide a low-solvent mixture,

where the low-solvent mixture does not form micelles that incorporate all of the hydrophobic drug, however when the low-solvent mixture is combined with additional biocompatible diblock copolymer (X-Y) having a hydrophilic block X comprising residues of monomer x and a hydrophobic block Y comprising residues of monomer y, then the resulting products does form micelles that incorporate all of the hydrophobic drug.

98. A composition formed by

(a) combining organic solvent, hydrophobic drug, biocompatible diblock copolymer (X-Y) having a hydrophilic block X comprising residues of monomer x and a hydrophobic block Y comprising residues of monomer y, and an additive selected from a polymer and a water soluble biocompatible organic liquid, to form a homogeneous

solution; and
(b) removing some or all of the organic solvent from the solution of (a) to provide a low-solvent mixture,

where the low-solvent mixture does not form micelles that incorporate all of the hydrophobic drug, however when the low-solvent mixture is combined with additional additive, then the resulting products does form micelles that incorporate all of the hydrophobic drug.

99. A composition formed by

(a) combining organic solvent, hydrophobic drug, biocompatible diblock copolymer (X-Y) having a hydrophilic block X comprising residues of monomer x and a hydrophobic block Y comprising residues of monomer y, and an additive selected from a polymer and a water soluble biocompatible organic liquid, to form a homogeneous solution; and
(b) removing some or all of the organic solvent from the solution of (a) to provide a low-solvent mixture,

where the low-solvent mixture does not form micelles that incorporate all of the hydrophobic drug, however when the low-solvent mixture is combined with additional biocompatible diblock copolymer (X-Y) having a hydrophilic block X comprising residues of monomer x and a hydrophobic block Y comprising residues of monomer y, and additional additive, then the resulting products does form micelles that incorporate all of the hydrophobic drug.

100. A method of forming a composition comprising

(a) combining solid hydrophobic drug and solid micelle-forming biocompatible diblock copolymer (X-Y) having a hydrophilic block X comprising residues of monomer x and a hydrophobic block y comprising residues of monomer y to provide a mixture;
(b) heating the mixture (a) to form a solution;
(c) cooling the solution (b) to provide a solid;
(d) milling or pulverizing the solid (c) to provide a powder.

101. The method of clause 100 further comprising

(e) heating the powder (d) to form a solution (e)
(f) cooling the solution (e) to provide a solid;
(g) milling or pulverizing the solid (e) to provide a powder.

102. The method of clause 101 wherein the drug is paclitaxel, block X is a polyether and block Y is a polyester.

103. A composition comprising solid hydrophobic drug and solid micelle-forming biocompatible diblock copolymer (X-Y) having a hydrophilic block X comprising residues of monomer x and a hydrophobic block y comprising residues of monomer y.

104. The composition of clause 103 wherein the hydrophobic drug is paclitaxel, block X is a polyether and block Y is a polyester.

105. A low organic solvent containing composition comprising hydrophobic drug and micelle-forming biocompatible diblock copolymer (X-Y) having a hydrophilic block X comprising residues of monomer x and a hydrophobic block y comprising residues of monomer y, wherein the solvent content of the composition is less than 1000 ppm.

106. The composition of clause 105 wherein the solvent content of the composition is less than 100 ppm.

107. The composition of clause 105 wherein the hydrophobic drug is paclitaxel, block X is a polyether and block Y is a polyester.

108. A fluid composition comprising hydrophobic drug and micelle-forming biocompatible diblock copolymer (X-Y) having a hydrophilic block X comprising residues of monomer x and a hydrophobic block y comprising residues of monomer y.

109. The composition of clause 108 wherein the hydrophobic drug is paclitaxel, block X is a polyether and block Y

is a polyester.

110. A composition comprising:

(a) a micelle-forming biocompatible diblock copolymer (X-Y) having a hydrophilic block X comprising residues of monomer x, and a hydrophobic block Y comprising residues of monomer y, wherein X comprises polyether and Y comprises polyester; and
(b) residual monomer y, where polymerization of y provides hydrophobic block Y; and where residual monomer y comprises less than 5% of the amount of monomer y present in the block Y.

111. A composition comprising a micelle-forming biocompatible diblock copolymer (X-Y) having a hydrophilic block X comprising residues of monomer x, and a hydrophobic block Y comprising residues of monomer y, wherein X comprises polyether and Y comprises polyester, said composition having an acid value of less than 0.4 μmol/mg acid.

112. A composition according to clauses 11 or 112 wherein the polyether is methyl-terminated polyethylene glycol and the polyester is poly(lactide).

113. A composition according to clauses 111 or 112 further comprising paclitaxel.

114. A method for preparing a composition, the method comprising

(a) dissolving a micelle-forming biocompatible diblock copolymer (X-Y) having a hydrophilic block X comprising residues of monomer x, and a hydrophobic block Y comprising residues of monomer y in a purification solvent,
(b) precipitating or crystallizing the diblock copolymer from the purification solvent; and
(c) separating the diblock copolymer of (b) from the purification solvent.

115. A method for preparing a composition, the method comprising

(a) dissolving a micelle-forming biocompatible diblock copolymer (X-Y) having a hydrophilic block X comprising residues of monomer x, and a hydrophobic block Y comprising residues of monomer y in a purification solvent,
(b) separating the purification solvent from the diblock copolymer by a physical separation technique selected from filtration and centrifugation;
(c) repeating (a); and
(d) repeating (b).

116. The method of clauses 114 or 115 wherein the diblock copolymer comprises a hydrophobic polyester block and a hydrophilic polyether block.

117. The method of clauses 114 or 115 wherein the purification solvent is selected from dichloromethane, ethanol, isopropanol, tetrahydrofuran, chloroform, and solvent mixtures including at least one of dichloromethane, ethanol, isopropanol, tetrahydrofuran, chloroform.

118. The method of clauses 114 or 115 wherein prior to (b), activated carbon is added to (a) and then the carbon is removed from (a) by filtration or centrifutation.

119. The method of clauses 114 or 115 wherein separating diblock copolymer from the purification solvent is conducted within one of a vacuum oven or forced-air oven or a vented oven.

120. A method of treating a disease in a mammal comprising the administration of an effective amount of a composition of clauses 1-76 to said mammal, said drug being efficacious at treating said disease.

121. A method of preventing a disease in a mammal comprising the administration of an effective amount of a composition of clauses 1-76 to said mammal, said drug being efficacious at preventing said disease.

122. A method of treating a disease in a mammal comprising the administration of an effective amount of a composition of clauses 78-82, 97-99 or 103-113 to said mammal.

123. A method of preventing a disease in a mammal comprising the administration of an effective amount of a

composition of clauses 78-82, 97-99 or 103-113 to said mammal.

124. A method of clause 120 wherein the disease is selected from inflammatory conditions, neurological disorders, cancer, and benign hyperproliferative diseases.

125. A method of clause 124 wherein the disease is arthritis.

126. A method of clause 124 wherein the disease is multiple sclerosis.

127. The method of clause 124 wherein the disease is Alzheimer's disease.

128. The method of clause 124 wherein the disease is psoriasis.

129. The method of clause 124 wherein the disease is cancer.

130. The method of clause 124 wherein the disease is stenosis or restenosis.

131. The method of clause 124 wherein the disease is benign hyperplasia.

132. The method of clause 131 wherein the hyperplasia is induced by a foreign body.

133. The method of clause 124 wherein the disease is cardiovascular disease.

134. The method of clause 124 wherein the disease is Inflammatory Bowel Disease.

135. The method of clause 120 wherein the composition is administered by a route selected from intravenous, intraarticular, intracutaneous, interstitial, subcutaneous, intramuscular injection, insertion into the rectum, oral, implant into the body.

136. The method of clause 135 wherein the composition is administered by intravenous delivery of an aqueous micelle solution.

137. The method of clause 135 wherein the composition is administered by implanting a semi-solid composition in the body, where the semi-solid composition gradually delivers drug-containing micelles to the body.

138. The method of clause 136 wherein the composition delivers paclitaxel or an analog or derivative thereof to the body of the mammal.

139. The method of clause 137 wherein the composition delivers paclitaxel or an analog or derivative thereof to the body of the mammal.

140. A method of clause 121 wherein the disease is selected from inflammatory conditions, neurological disorders, cancer, and benign hyperproliferative diseases.

141. A method of clause 140 wherein the disease is arthritis.

142. A method of clause 140 wherein the disease is multiple sclerosis.

143. The method of clause 140 wherein the disease is Alzheimer's disease.

144. The method of clause 140 wherein the disease is psoriasis.

145. The method of clause 140 wherein the disease is cancer.

146. The method of clause 140 wherein the disease is stenosis or restenosis.

147. The method of clause 140 wherein the disease is benign hyperplasia.

148. The method of clause 140 wherein the hyperplasia is induced by a foreign body.

149. The method of clause 140 wherein the disease is cardiovascular disease.

150. The method of clause 140 wherein the disease is Inflammatory Bowel Disease.

151. The method of clause 121 wherein the composition is administered by a route selected from intravenous, intraarticular, intracutaneous, interstitial, subcutaneous, intramuscular injection, insertion into the rectum, oral, implant into the body.

152. The method of clause 151 wherein the composition is administered by intravenous delivery of an aqueous micelle solution.

153. The method of clause 151 wherein the composition is administered by implanting a semi-solid composition in the body, where the semi-solid composition gradually delivers drug-containing micelles to the body.

154. The method of clause 152 wherein the composition delivers paclitaxel or an analog or derivative thereof to the body of the mammal.

155. The method of clause 153 wherein the composition delivers paclitaxel or an analog or derivative thereof to the body of the mammal.

156. A method of clause 122 wherein the disease is selected from inflammatory conditions, neurological disorders, cancer, and benign hyperproliferative diseases.

157. A method of clause 156 wherein the disease is arthritis.

158. A method of clause 156 wherein the disease is multiple sclerosis.

159. The method of clause 156 wherein the disease is Alzheimer’s disease.

160. The method of clause 156 wherein the disease is psoriasis.

161. The method of clause 156 wherein the disease is cancer.

162. The method of clause 156 wherein the disease is stenosis or restenosis.

163. The method of clause 156 wherein the disease is benign hyperplasia.

164. The method of clause 163 wherein the hyperplasia is induced by a foreign body.

165. The method of clause 156 wherein the disease is cardiovascular disease.

166. The method of clause 156 wherein the disease is Inflammatory Bowel Disease.

167. The method of clause 122 wherein the composition is administered by a route selected from intravenous, intraarticular, intracutaneous, interstitial, subcutaneous, intramuscular injection, insertion into the rectum, oral, implant into the body.

168. The method of clause 167 wherein the composition is administered by intravenous delivery of an aqueous micelle solution.

169. The method of clause 167 wherein the composition is administered by implanting a semi-solid composition in the body, where the semi-solid composition gradually delivers drug-containing micelles to the body.

170. The method of clause 168 wherein the composition delivers paclitaxel or an analog or derivative thereof to the body of the mammal.

171. The method of clause 169 wherein the composition delivers paclitaxel or an analog or derivative thereof to the body of the mammal.

172. A method of clause 123 wherein the disease is selected from inflammatory conditions, neurological disorders, cancer, and benign hyperproliferative diseases.

173. A method of clause 172 wherein the disease is arthritis.

174. A method of clause 124 wherein the disease is multiple sclerosis.

175. The method of clause 172 wherein the disease is Alzheimer’s disease.

176. The method of clause 172 wherein the disease is psoriasis.

177. The method of clause 172 wherein the disease is cancer.

178. The method of clause 172 wherein the disease is stenosis or restenosis.

179. The method of clause 172 wherein the disease is benign hyperplasia.

180. The method of clause 179 wherein the hyperplasia is induced by a foreign body.

181. The method of clause 172 wherein the disease is cardiovascular disease.

182. The method of clause 172 wherein the disease is Inflammatory Bowel Disease.

183. The method of clause 123 wherein the composition is administered by a route selected from intravenous, intraarticular, intracutaneous, interstitial, subcutaneous, intramuscular injection, insertion into the rectum, oral, implant into the body.

184. The method of clause 183 wherein the composition is administered by intravenous delivery of an aqueous micelle solution.

185. The method of clause 183 wherein the composition is administered by implanting a semi-solid composition in the body, where the semi-solid composition gradually delivers drug-containing micelles to the body.

186. The method of clause 184 wherein the composition delivers paclitaxel or an analog or derivative thereof to the body of the mammal.

187. The method of clause 185 wherein the composition delivers paclitaxel or an analog or derivative thereof to the body of the mammal.

188. A method of forming a composition comprising sequentially combining

(a) a micelle-forming biocompatible diblock copolymer (X-Y) having a hydrophilic block X comprising residues of monomer x, and a hydrophobic block Y comprising residues of monomer y;
(b) a hydrophobic drug;
(c) organic (processing) solvent ; and

removing the organic (processing) solvent by evaporation or distillation.

189. A method according to clause 188wherein the organic (processing) solvent comprises a solvent selected from tetrahydrofuran, ethanol, acetonitrile, chloroform, and dichloromethane.

190. A method according to clause 188 wherein the method further comprises heating the mixture to between 40-100°C to allow mixing and/or organic solvent removal.

## Claims

1. A composition comprising:

   (a) a micelle-forming biocompatible diblock copolymer (X-Y) having a hydrophilic block X, and a hydrophobic block Y;
   (b) a hydrophilic polymer additive wherein the hydrophilic polymer additive is poly(ethylene oxide) or the terminal $C_1$-$C_6$alkyl ethers thereof; and
   (c) a hydrophobic drug.

   wherein X comprises residues of alkylene oxide, Y comprises residues of monomers selected from lactic acid and reactive equivalents thereof, glycolic acid and reactive equivalents thereof, and caprylic acid and reactive equivalents thereof, wherein the composition comprises 1-15 parts diblock copolymer per each 1 part hydrophilic polymer additive; and wherein the composition is a lyophilized powder.

2. The composition of claim 1 wherein X comprises residues of ethylene oxide while Y comprises residues of lactide.

3. The composition of claim 1 wherein X is MePEG and Y is poly(DL-lactide).

4. The composition of claim 1 wherein the hydrophilic polymer additive is MePEG.

5. The composition of claim 4 wherein the MePEG has a molecular weight of 750 - 5000 g/mol.

6. The composition of claim 1 comprising about 10 parts diblock copolymer per each 1 part polymer.

7. The composition of claim 1 wherein the hydrophobic drug is selected from paclitaxel, paclitaxel derivatives and paclitaxel analogs.

8. The composition of claim 1 wherein the hydrophobic drug is paclitaxel.

9. The method of claim 1 further comprising a buffering constituent.

10. The composition of claim 9 wherein the buffering constituent comprises a phosphate salt.

11. The composition of claim 1 comprising, by weight, 10-90 parts diblock copolymer, 10-70 parts hydrophilic polymer additive, 1-15 parts paclitaxel and 1-20 parts phosphate salt.

12. The composition of claim 1 having less than 5% moisture content.

13. A method for forming a drug delivery vehicle, comprising sequentially

    (a) providing a composition according to any of claims 1-10; and
    (b) adding water to the composition of part (a) to form a micelle-containing composition.

14. A method of forming a composition of claim 1 comprising sequentially:

    combining the diblock copolymer, the hydrophilic polymer additive and the hydrophobic drug with an additional organic processing solvent;
    removing the organic processing solvent by evaporation or distillation to provide a mixture of the diblock copolymer, the hydrophilic polymer additive and hydrophobic drug;
    combining the mixture with an aqueous medium to provide a micellar solution; and
    lyophilizing the micellar solution.

15. A composition prepared according to claim 13 for use in a method of treating or preventing a disease in a mammal comprising the administration of an effective amount of said composition in an effective amount to said mammal, said drug being efficacious at treating said disease, the disease being inflammatory conditions, neurological disorders, cancer, or benign hyperproliferative diseases.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6096338 A
- US 6077543 A
- US 5843891 A
- US 5834019 A
- US 5827541 A
- US 5776486 A
- US 5645856 A
- US 5478860 A
- US 5430021 A
- US 032215 P
- US 60063087 P
- US 60275725 P
- US 60288017 P
- US 337935 P
- US 08094536 P
- US 08417160 P
- US 08480260 P
- US 08486867 P
- US 08980549 P
- US 08984258 P
- US 09013765 P
- US 09088546 P
- US 09368871 P
- US 09201695 P
- US 09294458 P
- US 09368463 P
- US 5716981 A
- US 5886026 A
- US 5994341 A
- EP 96119361 A
- EP 97945697 A
- EP 00123557 A
- EP 00123534 A
- EP 00123537 A
- EP 0706376 A
- CA 9400373 W
- CA 9700910 W
- CA 9900464 W
- WO 9407882 A
- WO 9407881 A
- WO 9407880 A
- WO 9407876 A
- WO 9323555 A
- WO 9310076 A
- WO 9400156 A
- WO 9324476 A
- EP 590267 A
- WO 9420089 A
- US 5294637 A
- US 5283253 A
- US 5279949 A
- US 5274137 A
- US 5202448 A
- US 5200534 A
- US 5229529 A
- US 5254580 A
- US 5412092 A
- US 5395850 A
- US 5380751 A
- US 5350866 A
- US 4857653 A
- US 5272171 A
- US 5411984 A
- US 5248796 A
- US 5422364 A
- US 5300638 A
- US 5362831 A
- US 5440056 A
- US 4814470 A
- US 5278324 A
- US 5352805 A
- US 5059699 A
- US 4942184 A
- US 6107332 A

### Non-patent literature cited in the description


- **ZHANG, X. et al.** *International Journal of Pharmaceutics,* 1996, vol. 132, 195-206
- **WANI et al.** *J. Am. Chem. Soc.,* 1971, vol. 93, 2325
- **STIERLE et al.** *Science,* 1993, vol. 60, 214-216
- **SCHIFF et al.** *Nature,* 1979, vol. 277, 665-667
- **LONG ; FAIRCHILD.** *Cancer Research,* 1994, vol. 54, 4355-4361
- **RINGEL ; HORWITZ.** *J. Nat'l Cancer Inst.,* 1991, vol. 83 (4), 288-291
- **PAZDUR et al.** *Cancer Treat. Rev.,* 1993, vol. 19 (4), 351-386
- *Tetrahedron Letters,* 1994, vol. 35 (52), 9709-9712

- *J. Med. Chem.,* 1992, vol. 35, 4230-4237
- *J. Med. Chem.,* 1991, vol. 34, 992-998
- *J. Natural Prod.,* 1994, vol. 57 (10), 1404-1410
- *J. Natural Prod.,* 1994, vol. 57 (11), 1580-1583
- *J. Am. Chem. Soc.,* 1988, vol. 110, 6558-6560
- Remingtons Pharmaceutical Sciences. Mack Publishing Co, 1985
- Handbook of Applied Surface and Colloid Chemistry. John Wiley & Sons, 2001